# EUROPEAN PATENT APPLICATION

(11) **EP 2 380 515 A1**
(43) Date of publication of application: **26.10.2011**
(21) Application number: 09838873.9
(22) Date of filing: 22.12.2009
(51) Int. Cl.: A61B 18/12

(54) **TREATMENT DEVICE AND TREATMENT TOOL**

(30) Priority: 21.01.2009 US 356767
(71) Applicant: OLYMPUS MEDICAL SYSTEMS CORP., Tokyo 151-0072 (JP)
(72) Inventor: SAKAO, Satomi, Tokyo 192-8512 (JP); IIDA, Koji, Tokyo 192-8512 (JP); INAGAKI, Genri, Tokyo 192-8512 (JP); TAKASHINO, Tomoyuki, Tokyo 192-8512 (JP)
(74) Representative: Schicker, Silvia
(86) International application number: PCT/JP2009/071275
(87) International publication number: WO 2010/084683

(57) **Abstract**

A medical treatment instrument (12) includes a first treating portion (52), a second treating portion (54) and an operation portion (32). The first treating portion includes holding members (62, 64) to hold the target living tissues and an energy output unit (82b, 84b) which join the target living tissues when the target living tissues is applied thereto from the energy source. The second treating portion is interposed between the target living tissues and denatures the surfaces of the target living tissues in contact with the second treating portion. The operation portion (32) has a function of operating the holding members so that the holding members moves relative to the other. The energy output unit and the second treating portion are able to detect at least one of biological information regarding the living tissues held by the holding members and biological information regarding the living tissues between the holding member and the second treating portion, and control energy output from the energy source to the energy output unit and the second treating portion is controlled on the basis of the biological information regarding the living tissues obtained by the energy output unit and the second treating portion.

## Description

### Technical Field

This invention relates to a medical treatment apparatus and a medical treatment instrument that are capable of joining a plurality of living tissues together using energy.

### Background Art

In surgical operations including an abdominal operation and a laparoscopic operation, a tubular tissue or organ of, for example, a blood vessel may be sealed, or other living tissues may be joined together. For example, a suture or clip is used to seal the blood vessel to be disjoined. A suture or staple is used to seal or anastomose cut ends of a digestive tract. In addition, techniques using energy have been in use recently. A high-frequency device or ultrasonic device is constantly used to seal a blood vessel, and moreover, other devices used for thicker tissues are also making progress.

A device for sealing and joining living tissues by energy fusion-bonds the living tissues with a grasping forceps having electrodes. Such a procedure requires application of energy to a part (joint) where the living tissues to be joined together are in contact with each other, and adequate denaturation and dehydration of the living tissues at the joint.

However, the problem in the case where, for example, living tissues having a certain degree of thickness are sealed and joined together is that sufficient energy is not input to joint surfaces of the living tissues located far from electrodes of a holding member. The cause of this problem is as follows: In, for example, the high-frequency device, the impedance around the living tissues in contact with the electrodes of the holding member rises before the impedance inside the living tissues, so that a treatment is ended before the impedance inside the living tissues rises enough. On the other hand, in the ultrasonic device, a treatment is performed by the conduction of frictional heat, so that the treatment is ended before the spread of heat from the surface reaches the inside of the living tissues. Therefore, sufficient energy may not be input to the living tissues having a certain degree of thickness.

As a solution, a bipolar device is disclosed in, for example, USP 6,500,176 B1. According to the disclosed technique of the bipolar device, an electrode is inserted into the centers of living tissues to be joined together, and electric energy is applied to a grasping forceps from the electrode between the living tissues. The bipolar device is thus capable of fusion-bonding and sealing thick living tissues and living tissues having various compositions.

Furthermore, Jpn. Pat. Appln. KOKAI Publication No. 2007-229270 discloses no forceps having high-frequency electrodes, but discloses a technique of directly applying energy to joint surfaces of living tissues to be joined together. According to the technique of Jpn. Pat. Appln. KOKAI Publication No. 2007-229270, an ultrasonic probe is placed on the joint surfaces of living tissues, and the ultrasonic probe is ultrasonically vibrated on the joint surfaces to heat and join the living tissues.

However, although USP 6,500,176 B1 discloses power supply from a central electrode to electrodes in a grasping portion only by a predetermined method, it does not disclose power supply by switching electrodes which output power in accordance with a state of the living tissues.

Further, according to the technique disclosed in Jpn. Pat. Appln. KOKAI Publication No. 2007-229270, the ultrasonic probe is disposed on joint surfaces of living tissues grasped by a grasping portion to apply vibrations and produce an increase in temperature. However, since there is no mechanism for switching outputs in accordance with a state of tissues, the tissues cannot be satisfactorily dehydrated or denatured.

### Disclosure of Invention

An object of the present invention is to provide a medical treatment apparatus and a medical treatment instrument configured to easily denature and dehydrate joint surfaces of living tissues by switching combinations of electrodes to efficiently concentrate energy on the joint surfaces, while monitoring a state of the living tissues.

According to a first aspect of the present invention, there is provided a medical treatment apparatus to join target living tissues in a body, the medical treatment apparatus including: an energy source which applies energy to the target living tissues; a first treating portion which joins the target living tissues together when energy is applied thereto from the energy source; a second treating portion which is interposed between the target living tissues and which denatures the surfaces of the target living tissues in contact with the second treating portion; a detecting portion; and a controller. The first treating portion includes at least a pair of holding members having holding surfaces to hold the target living tissues, and an energy output unit which is provided on the holding surfaces of the holding members and which join the target living tissues together when energy is applied thereto from the energy source. The detecting portion detects at least one of biological information regarding the living tissues held by the pair of holding members and biological information regarding the living tissues between the pair of holding members and the second treating portion. The controller controls an output of the energy output unit and the second treating portion on the basis of the biological information regarding the living tissues obtained in the detecting portion.

According to a second aspect of the present invention, there is provided a medical treatment instrument to join target living tissues in a body, the treatment instrument including: a first treating portion which joins the target living tissues together when energy is applied thereto from an energy source; a second treating portion which is interposed between the target living tissues and which denatures the surfaces of the target living tissues in contact with the second treating portion; and an operation portion. The first treating portion includes at least a pair of holding members having holding surfaces to hold the target living tissues, and an energy output unit which is provided on the holding surfaces of the holding members and which join the target living tissues together when energy is applied thereto from the energy source. The operation portion has a function of operating the holding members so that at least one of the holding members moves relative to the other. The energy output unit and the second treating portion are able to detect at least one of biological information regarding the living tissues held by the pair of holding members and biological information regarding the living tissues between the holding member and the second treating portion. Energy output from the energy source to the energy output unit and the second treating portion is controlled on the basis of the biological information regarding the living tissues obtained in the energy output unit and the second treating portion.

### Brief Description of Drawings

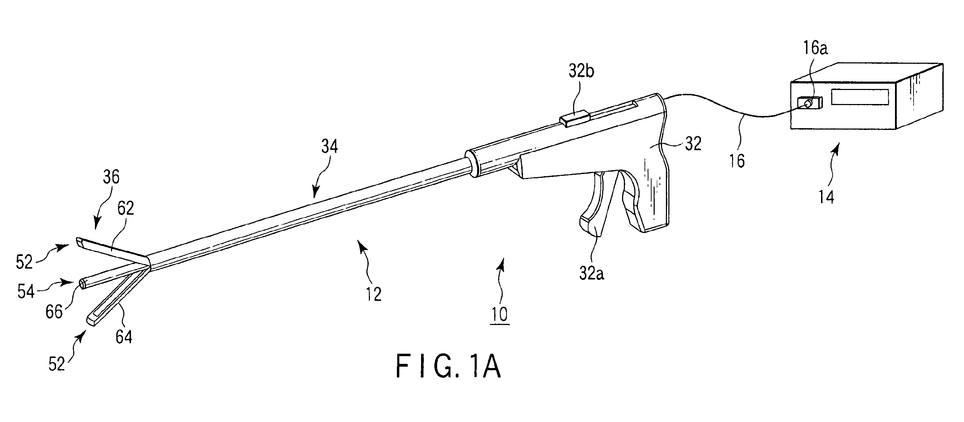

FIG. 1A is a schematic perspective view showing a medical treatment apparatus according to a first embodiment;

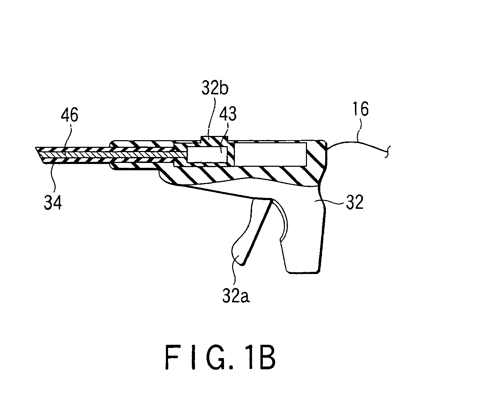

FIG. 1B is a partial sectional view of a handle and a shaft of an energy treatment instrument of the medical treatment apparatus according to the first embodiment;

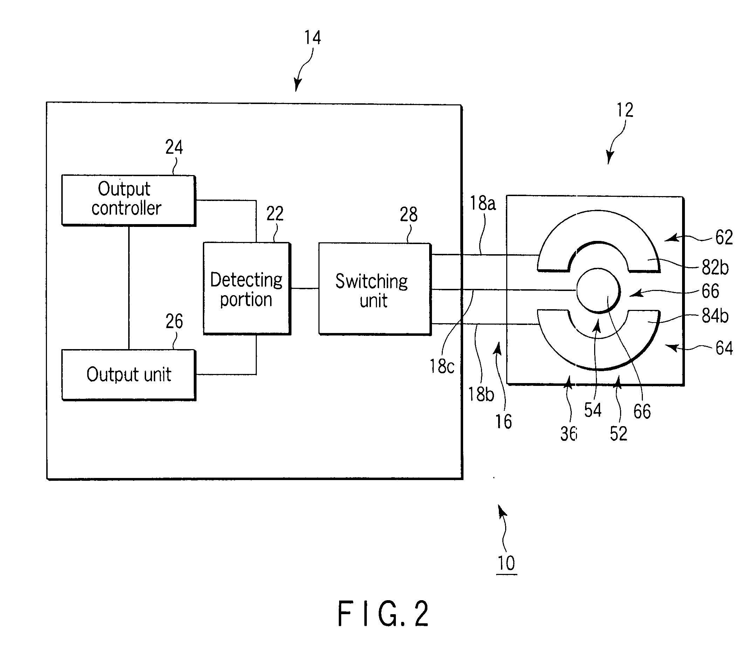

FIG. 2 is a schematic diagram showing the medical treatment apparatus according to the first embodiment;

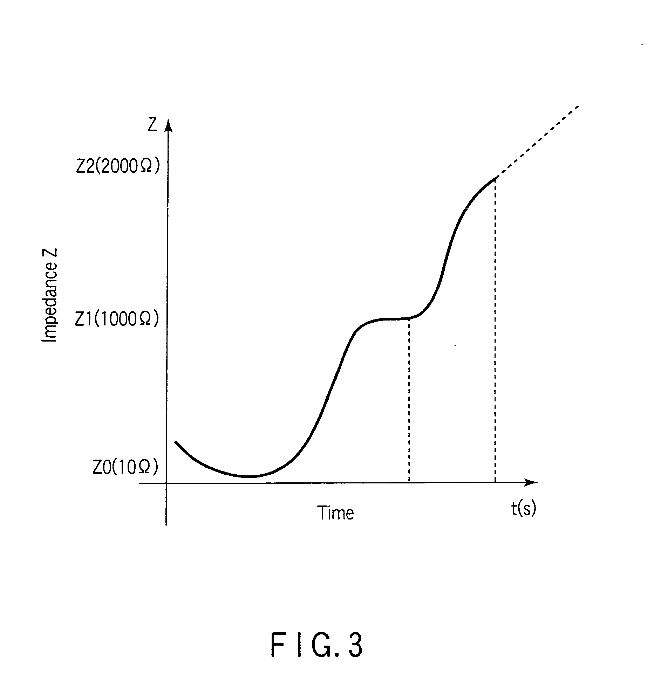

FIG. 3 is a schematic graph showing the relation of the impedance of living tissues with time when the medical treatment apparatus is used to continuously apply high-frequency energy to living tissues and thereby treat the living tissues;

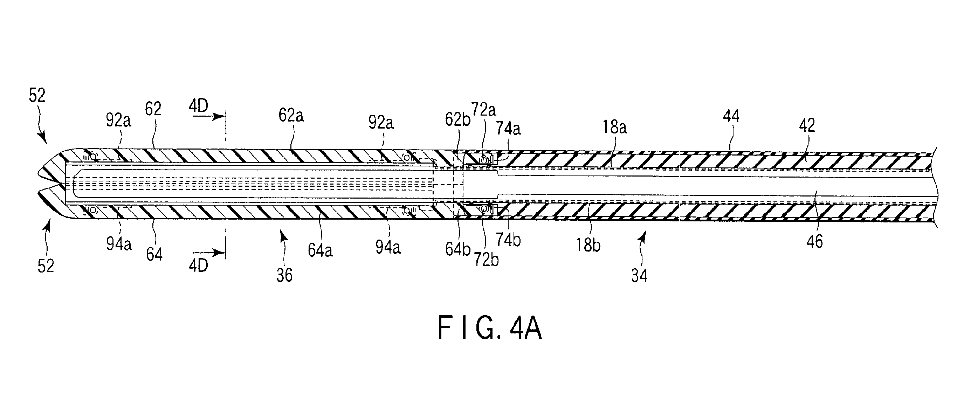

FIG. 4A is a schematic longitudinal sectional view showing the shaft and a treatment portion in which first and second holding members are closed and in which a rod electrode is disposed between the first and second holding members of the energy treatment instrument of the medical treatment apparatus according to the first embodiment;

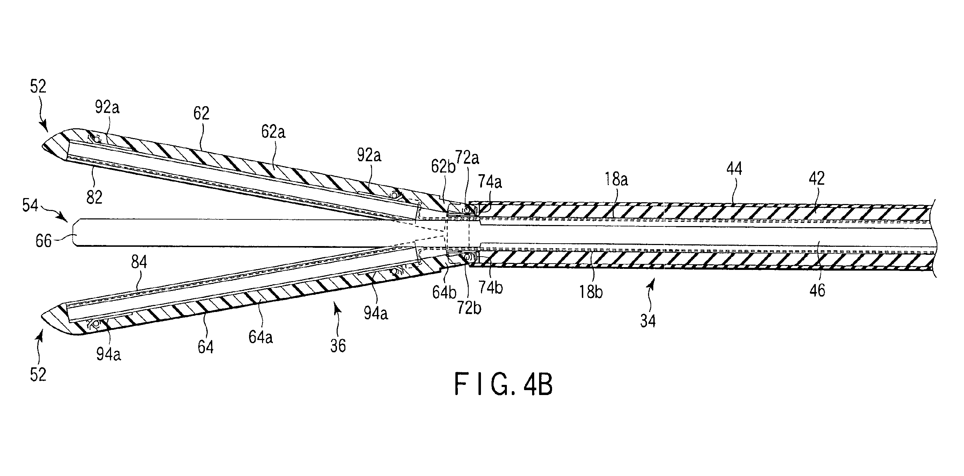

FIG. 4B is a schematic longitudinal sectional view showing the shaft and the treatment portion in which the first and second holding members are opened and in which the rod electrode is disposed between the first and second holding members of the energy treatment instrument of the medical treatment apparatus according to the first embodiment;

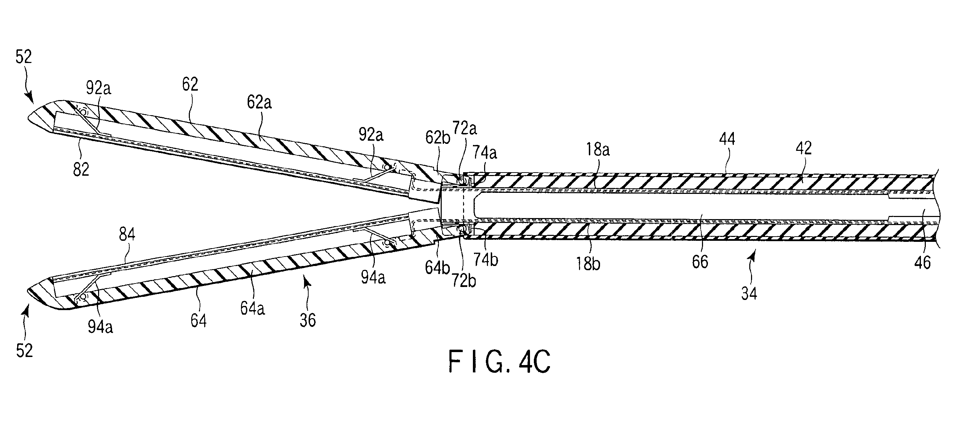

FIG. 4C is a schematic longitudinal sectional view showing the shaft and the treatment portion in which the first and second holding members are opened and in which the rod electrode is drawn into the shaft from between the first and second holding members of the energy treatment instrument of the medical treatment apparatus according to the first embodiment;

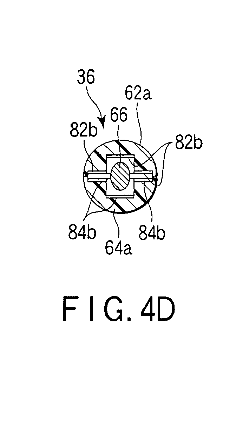

FIG. 4D is a schematic cross sectional view along the line 4D-4D in FIG. 4A, wherein the first and second holding members of the treatment portion are closed, and the rod electrode is disposed between the first and second holding members of the energy treatment instrument of the medical treatment apparatus according to the first embodiment;

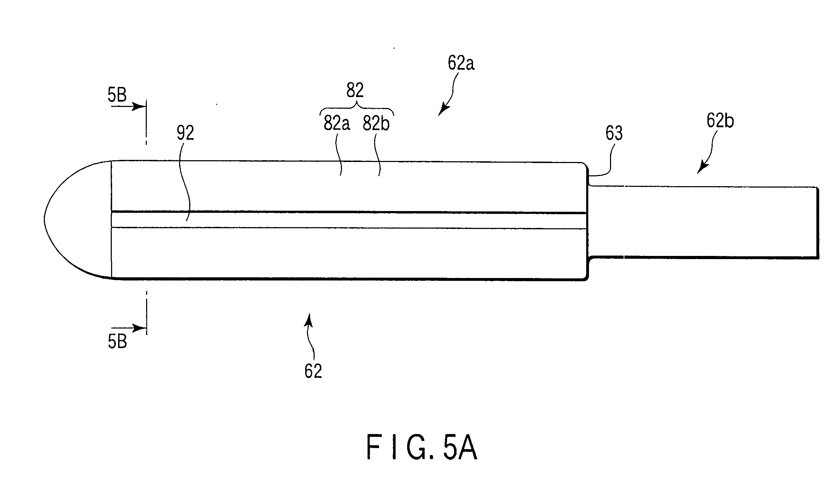

FIG. 5A is a schematic view showing a holding surface of the main body of the first holding member of the treatment portion of the energy treatment instrument of the medical treatment apparatus according to the first embodiment;

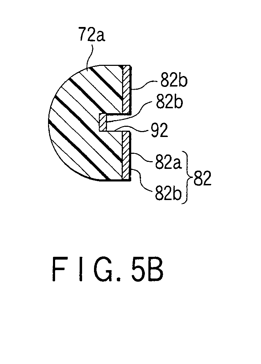

FIG. 5B is a schematic cross sectional view along the line 5B-5B in FIG. 5A, showing the main body of the first holding member of the treatment portion of the energy treatment instrument of the medical treatment apparatus according to the first embodiment;

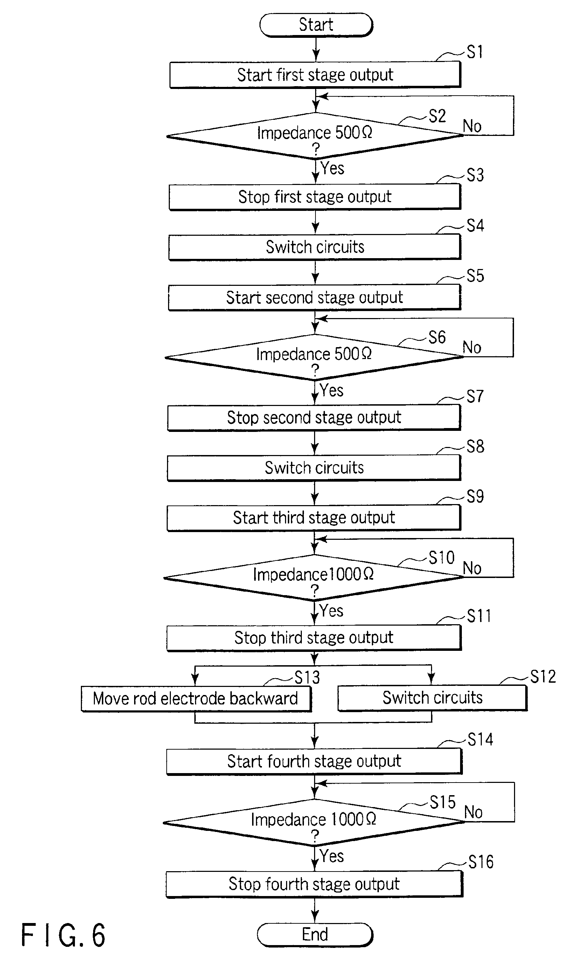

FIG. 6 is a flowchart for joining living tissues by use of the medical treatment apparatus according to the first embodiment;

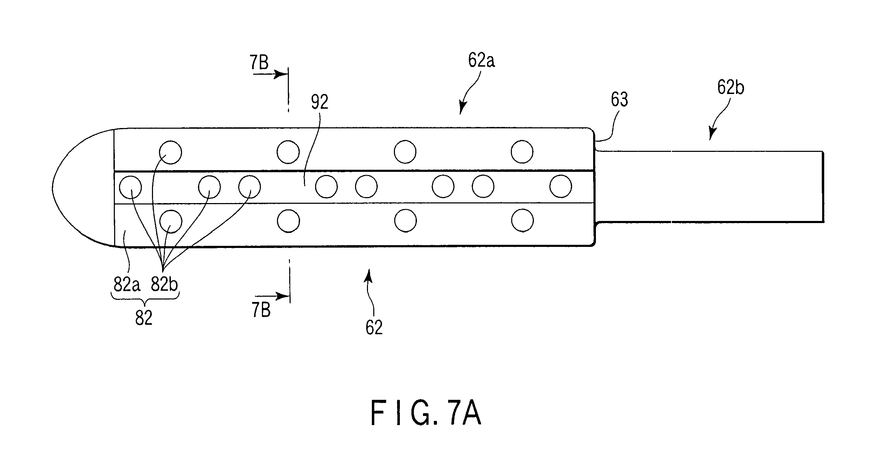

FIG. 7A is a schematic view showing the holding surface of the main body of the first holding member of the treatment portion of the energy treatment instrument of the medical treatment apparatus according to a modification of the first embodiment;

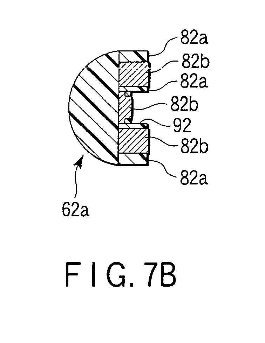

FIG. 7B is a schematic cross sectional view along the line 7B-7B in FIG. 7A, showing the main body of the first holding member of the treatment portion of the energy treatment instrument of the medical treatment apparatus according to the modification of the first embodiment;

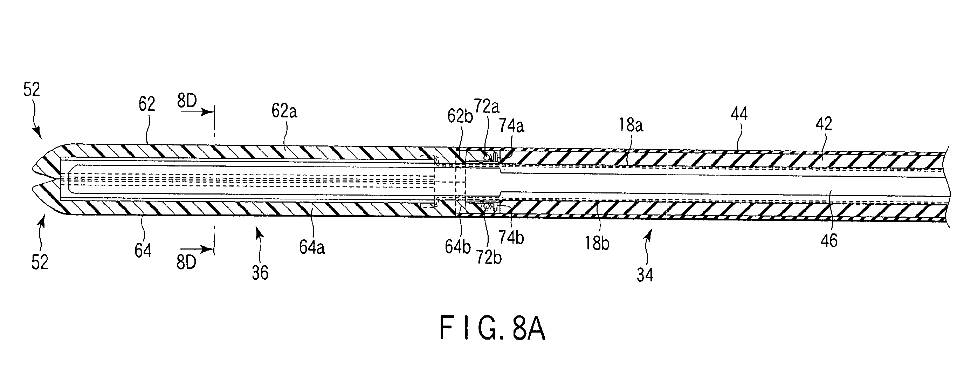

FIG. 8A is a schematic longitudinal sectional view showing the shaft and the treatment portion in which the first and second holding members are closed and in which the rod electrode is disposed between the first and second holding members of the energy treatment instrument of the medical treatment apparatus according to a modification of the first embodiment;

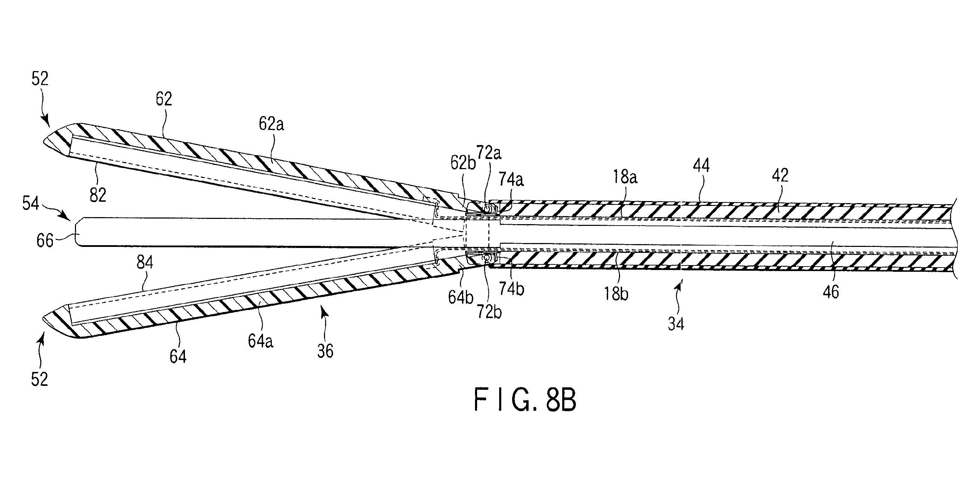

FIG. 8B is a schematic longitudinal sectional view showing the shaft and the treatment portion in which the first and second holding members are opened and in which the rod electrode is disposed between the first and second holding members of the energy treatment instrument of the medical treatment apparatus according to the modification of the first embodiment;

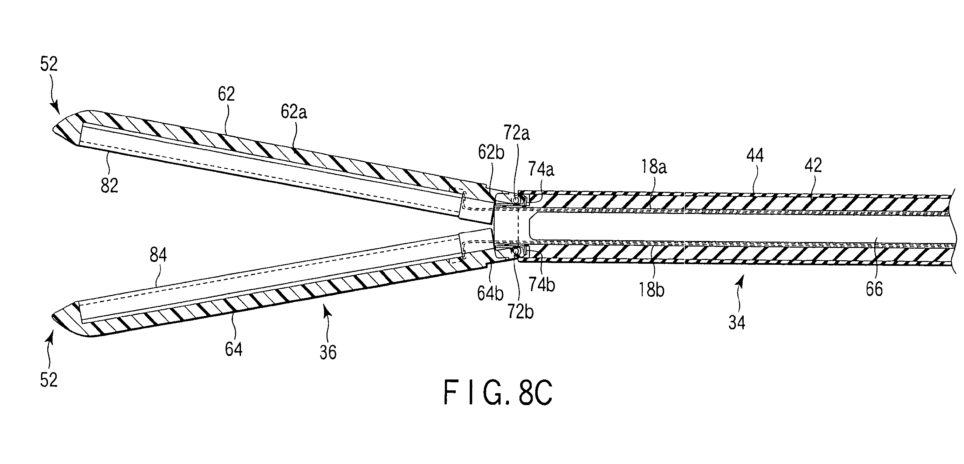

FIG. 8C is a schematic longitudinal sectional view showing the shaft and the treatment portion in which the first and second holding members are opened and in which the rod electrode is drawn into the shaft from between the first and second holding members of the energy treatment instrument of the medical treatment apparatus according to the modification of the first embodiment;

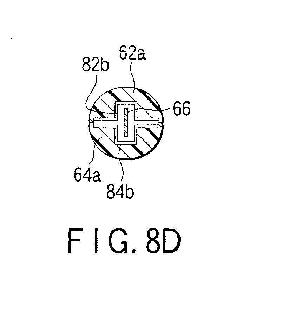

FIG. 8D is a schematic cross sectional view along the line 8D-8D in FIG. 8A, wherein the first and second holding members of the treatment portion are closed, and the rod electrode is disposed between the first and second holding members of the energy treatment instrument of the medical treatment apparatus according to the modification of the first embodiment;

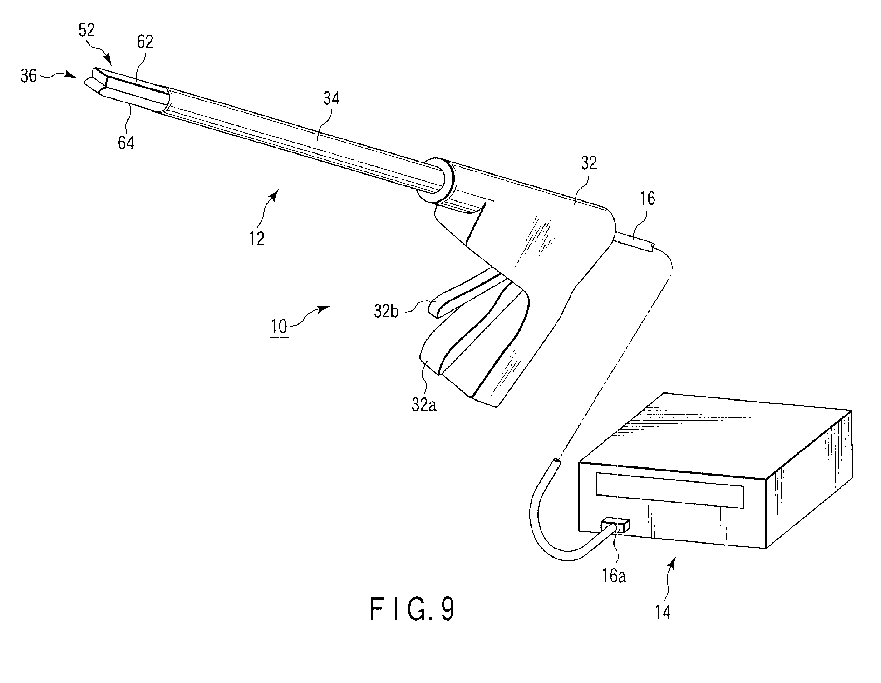

FIG. 9 is a schematic perspective view showing the medical treatment apparatus according to a modification of the first embodiment;

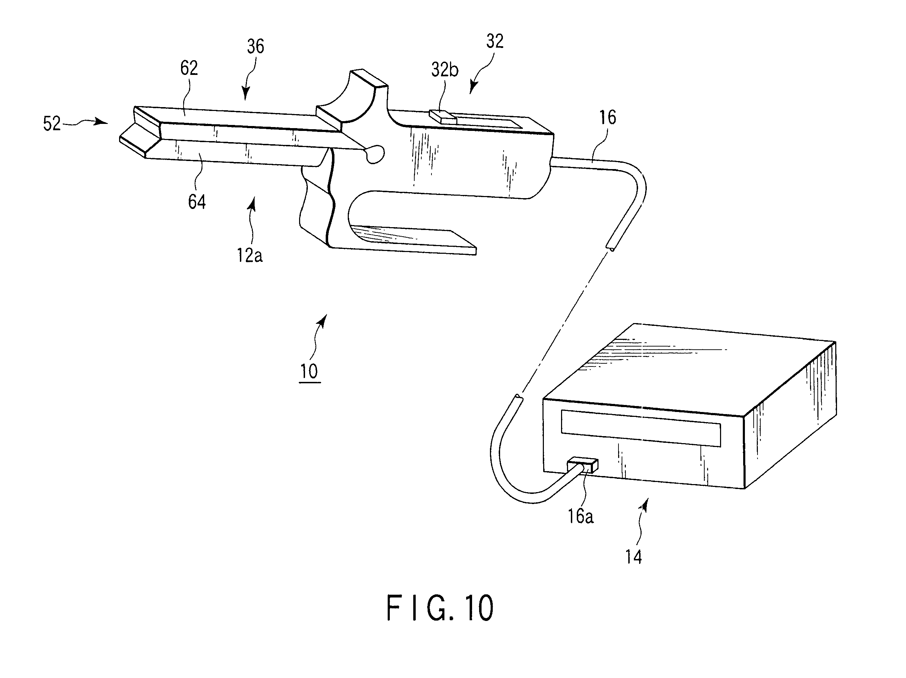

FIG. 10 is a schematic perspective view showing the medical treatment apparatus according to a modification of the first embodiment;

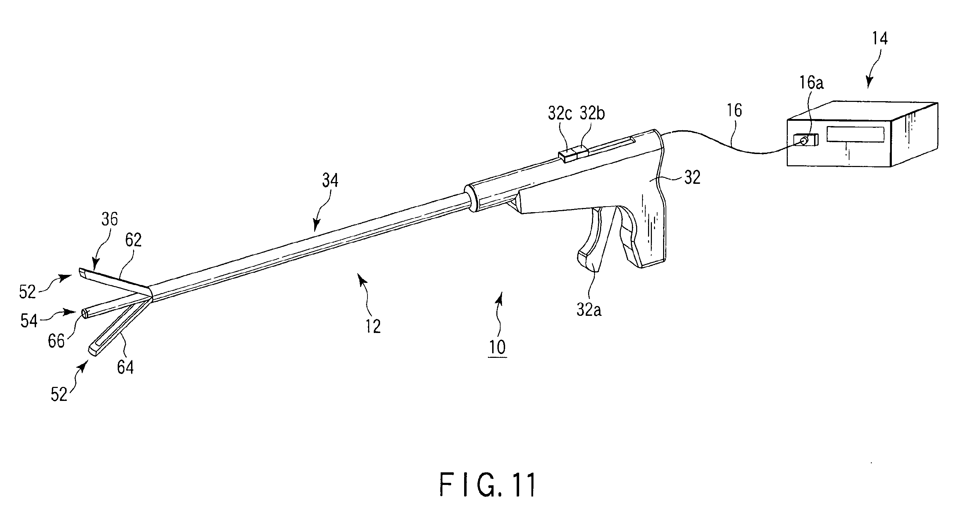

FIG. 11 is a schematic perspective view showing a medical treatment apparatus according to a second embodiment;

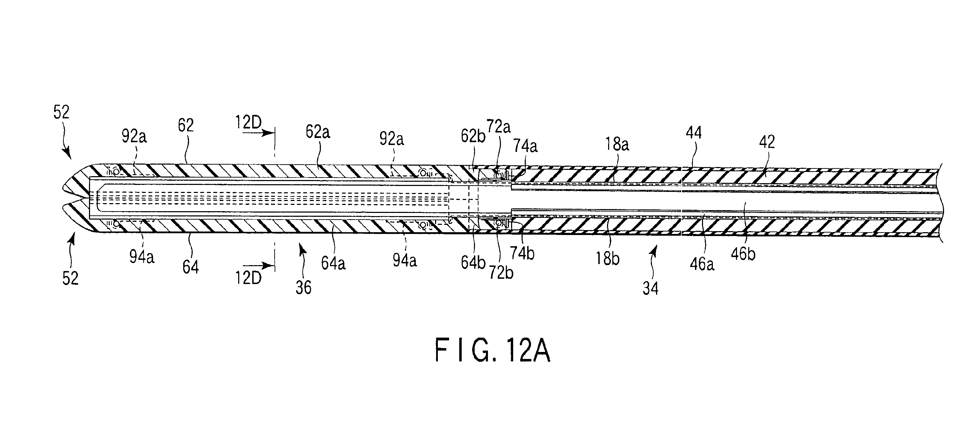

FIG. 12A is a schematic longitudinal sectional view showing a shaft and a treatment portion in which first and second holding members are closed and in which a rod electrode is disposed between the first and second holding members of an energy treatment instrument of the medical treatment apparatus according to the second embodiment;

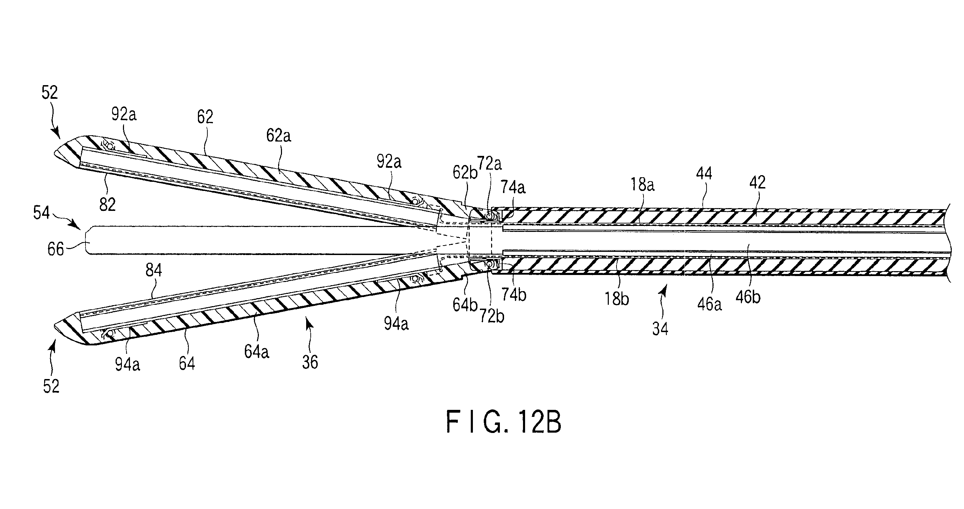

FIG. 12B is a schematic longitudinal sectional view showing the shaft and the treatment portion in which the first and second holding members are opened and in which the rod electrode is disposed between the first and second holding members of the energy treatment instrument of the medical treatment apparatus according to the second embodiment;

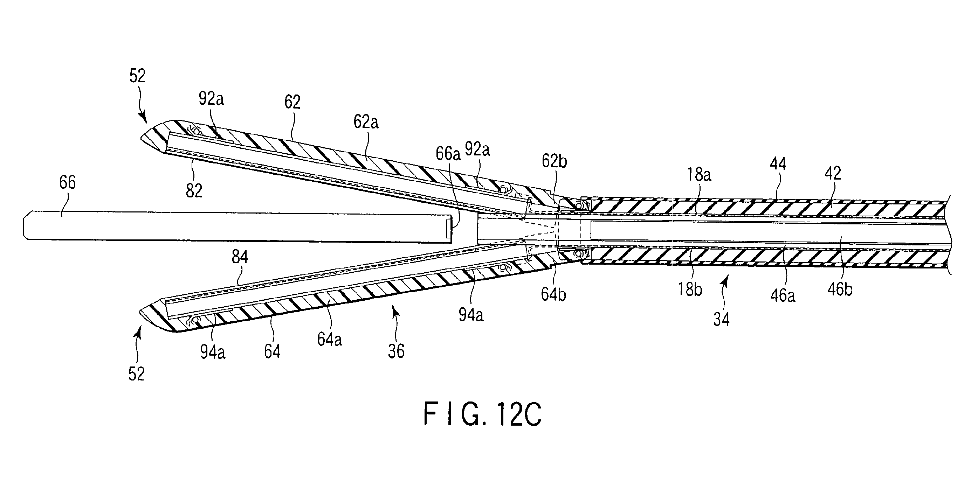

FIG. 12C is a schematic longitudinal sectional view showing the shaft and the treatment portion in which the first and second holding members are opened and in which the rod electrode is separated from between the first and second holding members of the energy treatment instrument of the medical treatment apparatus according to the second embodiment;

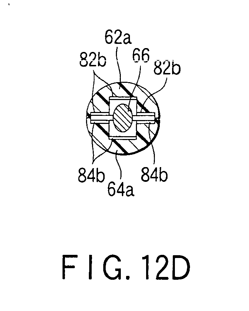

FIG. 12D is a schematic cross sectional view along the line 12D-12D in FIG. 12A, wherein the first and second holding members of the treatment portion are closed, and the rod electrode is disposed between the first and second holding members of the energy treatment instrument of the medical treatment apparatus according to the second embodiment;

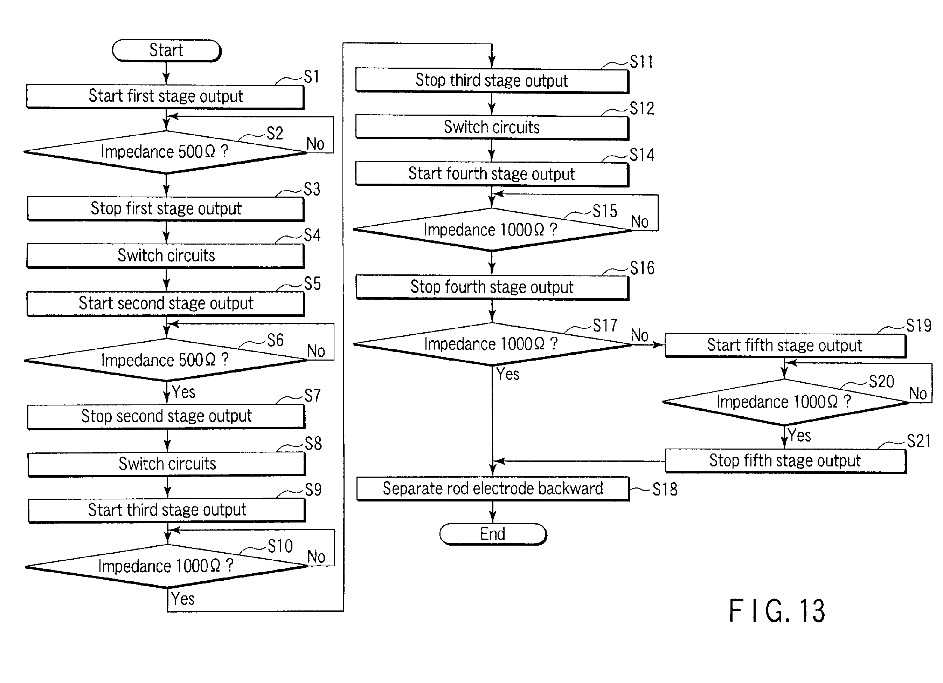

FIG. 13 a flowchart for joining living tissues by use of the medical treatment apparatus according to the second embodiment;

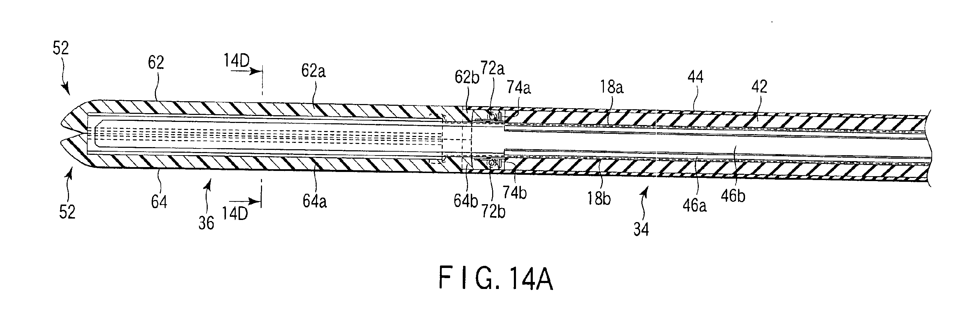

FIG. 14A is a schematic longitudinal sectional view showing the shaft and the treatment portion in which the first and second holding members are closed and in which the rod electrode is disposed between the first and second holding members of the energy treatment instrument of the medical treatment apparatus according to a modification of the second embodiment;

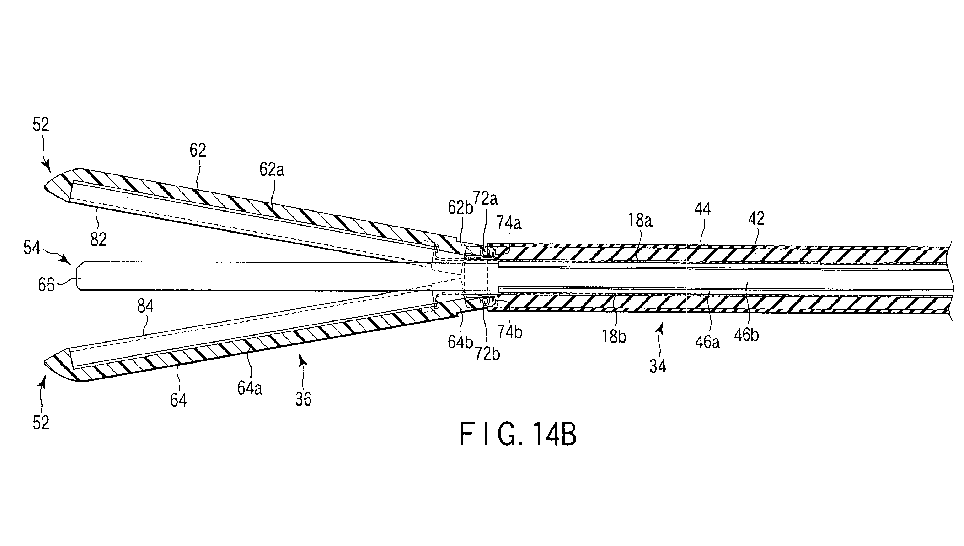

FIG. 14B is a schematic longitudinal sectional view showing the shaft and the treatment portion in which the first and second holding members are opened and in which the rod electrode is disposed between the first and second holding members of the energy treatment instrument of the medical treatment apparatus according to the modification of the second embodiment;

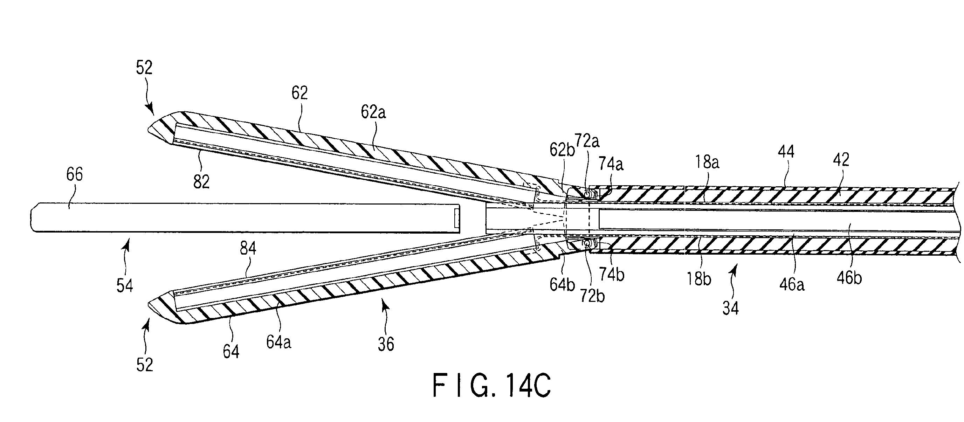

FIG. 14C is a schematic longitudinal sectional view showing the shaft and the treatment portion in which the first and second holding members are opened and in which the rod electrode is separated from between the first and second holding members of the energy treatment instrument of the medical treatment apparatus according to the modification of the second embodiment;

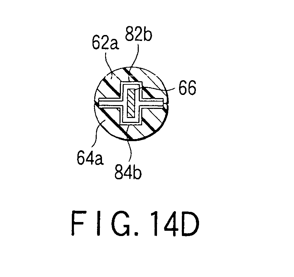

FIG. 14D is a schematic cross sectional view along the line 14D-14D in FIG. 14A, wherein the first and second holding members of the treatment portion are closed, and the rod electrode is disposed between the first and second holding members of the energy treatment instrument of the medical treatment apparatus according to the modification of the second embodiment;

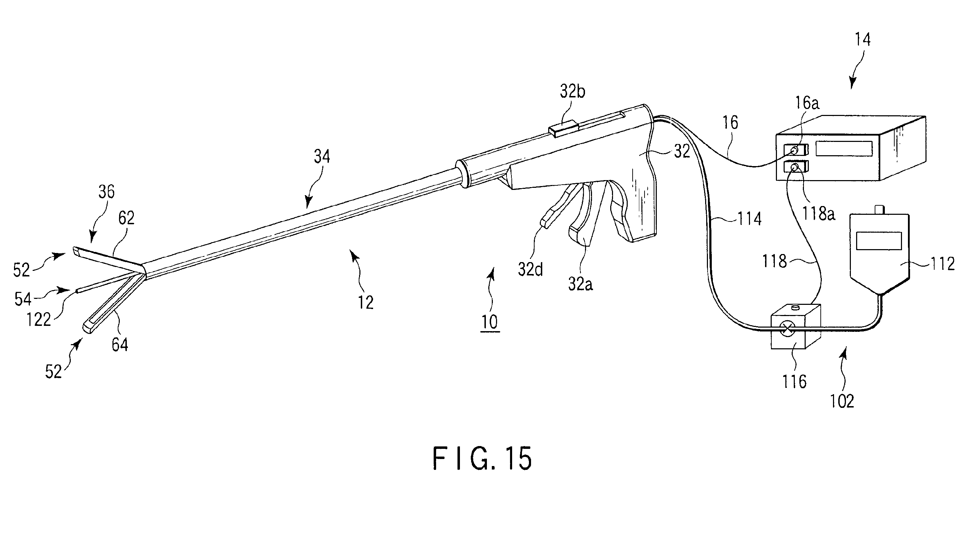

FIG. 15 is a schematic perspective view showing a medical treatment apparatus according to a third embodiment;

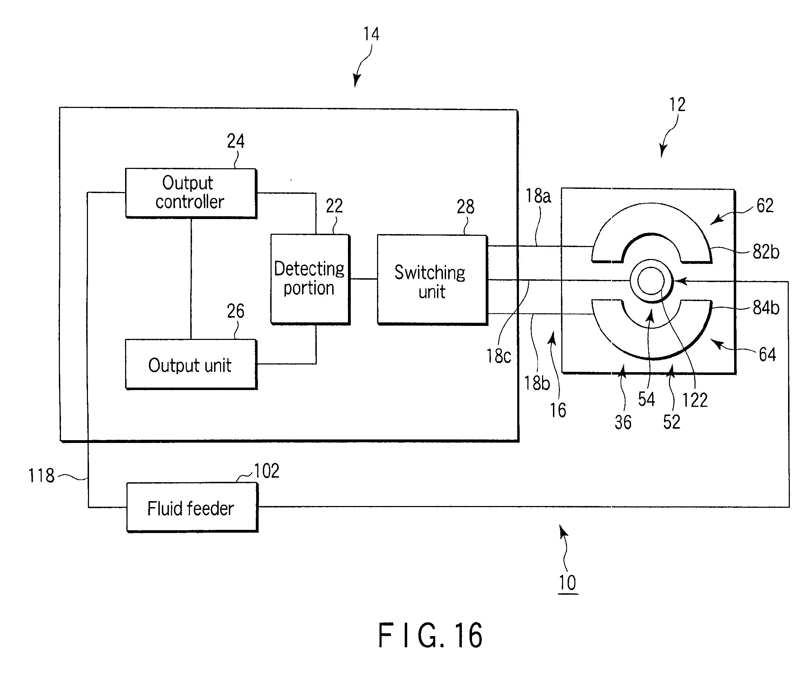

FIG. 16 is a schematic diagram showing the medical treatment apparatus according to the third embodiment;

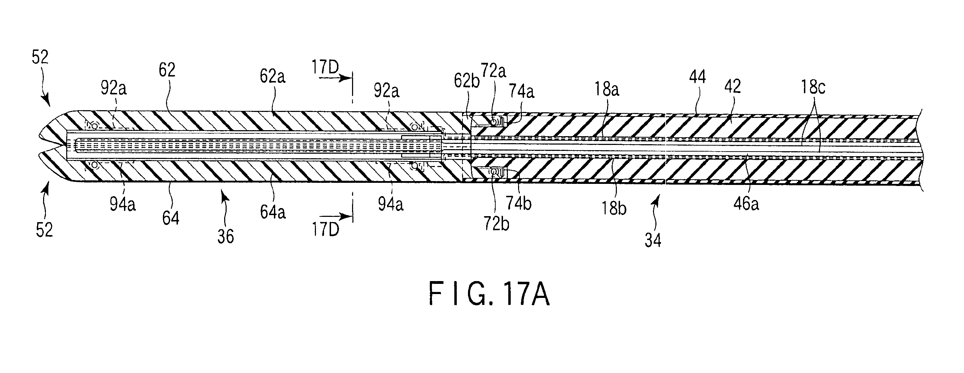

FIG. 17A is a schematic longitudinal sectional view showing a shaft and a treatment portion in which first and second holding members are closed and in which a tubular electrode is disposed between the first and second holding members of an energy treatment instrument of the medical treatment apparatus according to the third embodiment;

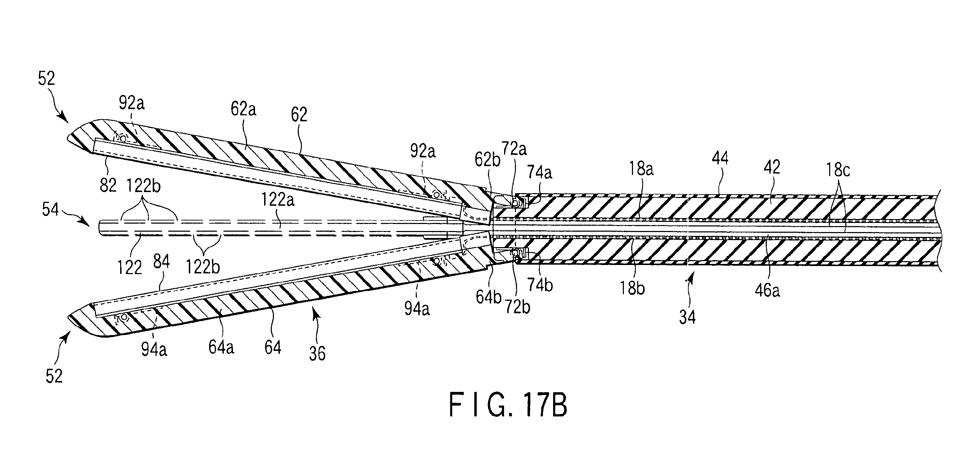

FIG. 17B is a schematic longitudinal sectional view showing the shaft and the treatment portion in which the first and second holding members are opened and in which the tubular electrode is disposed between the first and second holding members of the energy treatment instrument of the medical treatment apparatus according to the third embodiment;

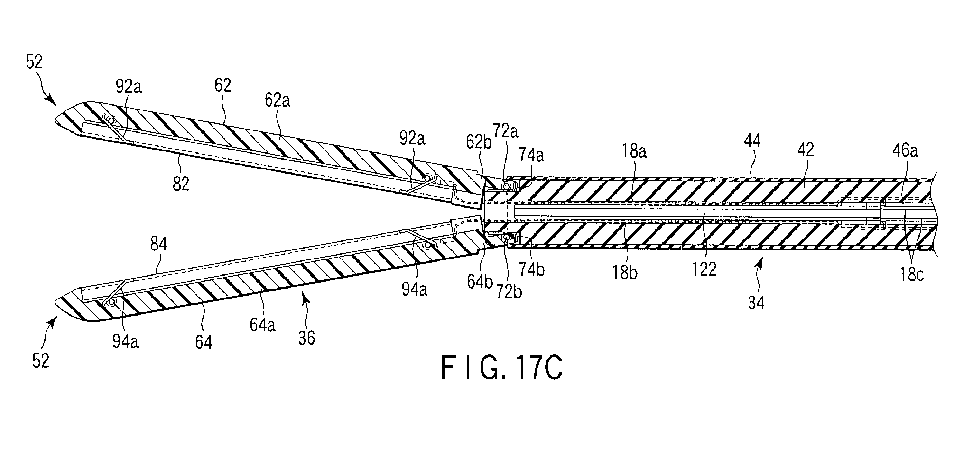

FIG. 17C is a schematic longitudinal sectional view showing the shaft and the treatment portion in which the first and second holding members are opened and in which the tubular electrode is drawn into the shaft from between the first and second holding members of the energy treatment instrument of the medical treatment apparatus according to the third embodiment;

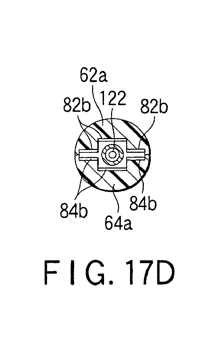

FIG. 17D is a schematic cross sectional view along the line 17D-17D in FIG. 17A, wherein the first and second holding members of the treatment portion are closed, and the tubular electrode is disposed between the first and second holding members of the energy treatment instrument of the medical treatment apparatus according to the third embodiment;

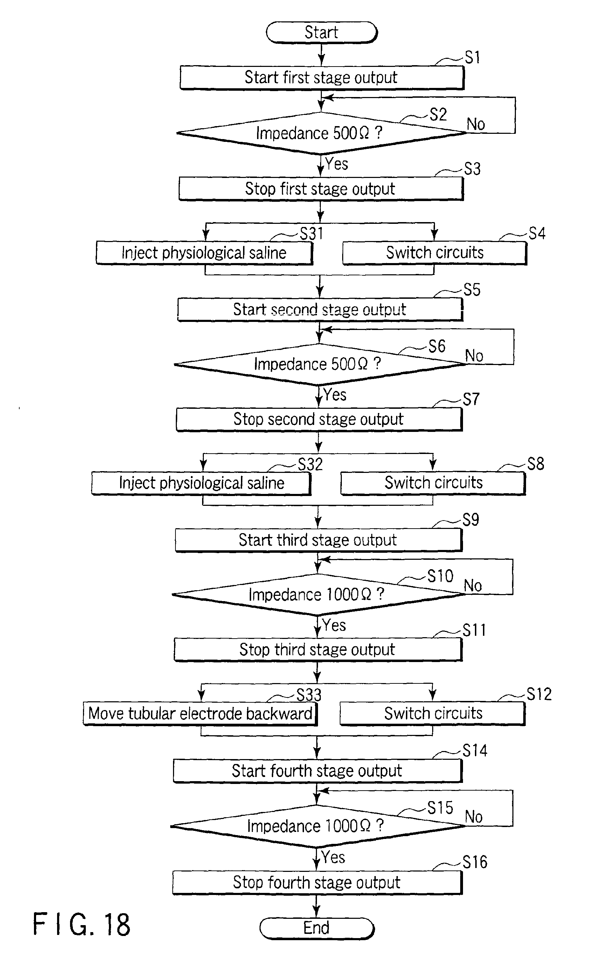

FIG. 18 a flowchart for joining living tissues by use of the medical treatment apparatus according to the third embodiment;

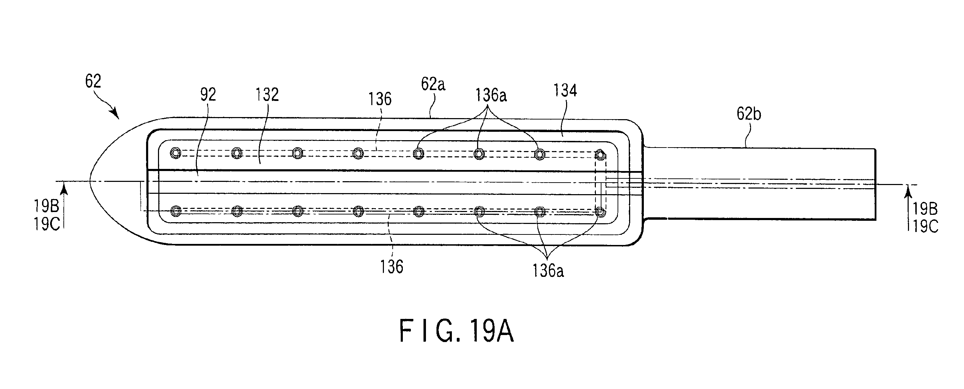

FIG. 19A is a schematic view showing a holding surface of the main body of a first holding member of a treatment portion of an energy treatment instrument in a medical treatment apparatus according to a fourth embodiment;

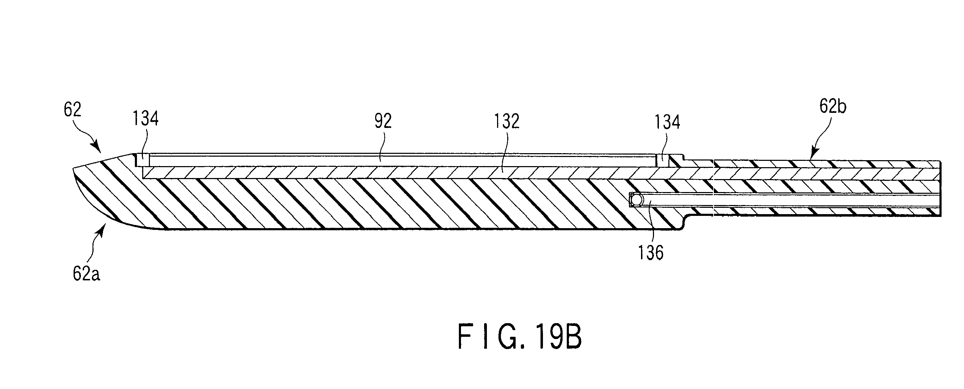

FIG. 19B is a schematic longitudinal sectional view along the line 19B-19B in FIG. 19A, showing the main body and a base of the first holding member of the treatment portion of the energy treatment instrument of the medical treatment apparatus according to the fourth embodiment;

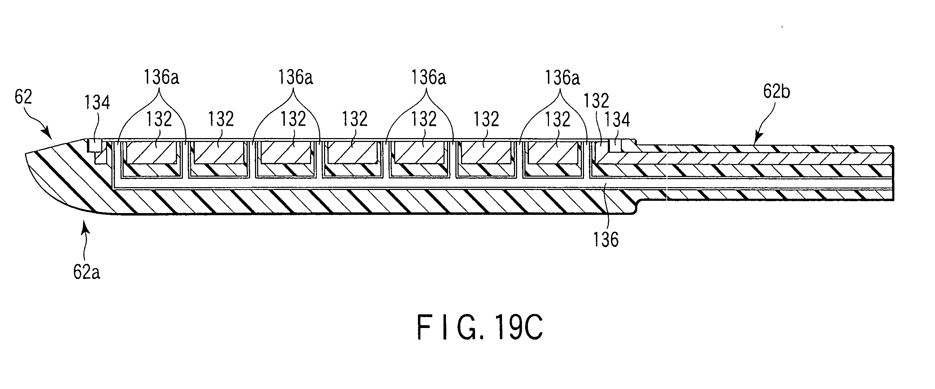

FIG. 19C is a schematic longitudinal sectional view along the line 19C-19C in FIG. 19A, showing the main body and the base of the first holding member of the treatment portion of the energy treatment instrument of the medical treatment apparatus according to the fourth embodiment;

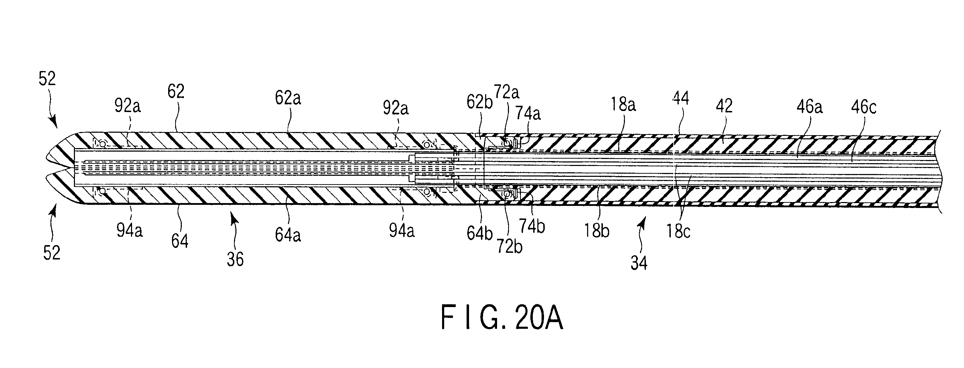

FIG. 20A is a schematic longitudinal sectional view showing a shaft and a treatment portion in which first and second holding members are closed and in which a tubular electrode is disposed between the first and second holding members of an energy treatment instrument in a medical treatment apparatus according to a fifth embodiment;

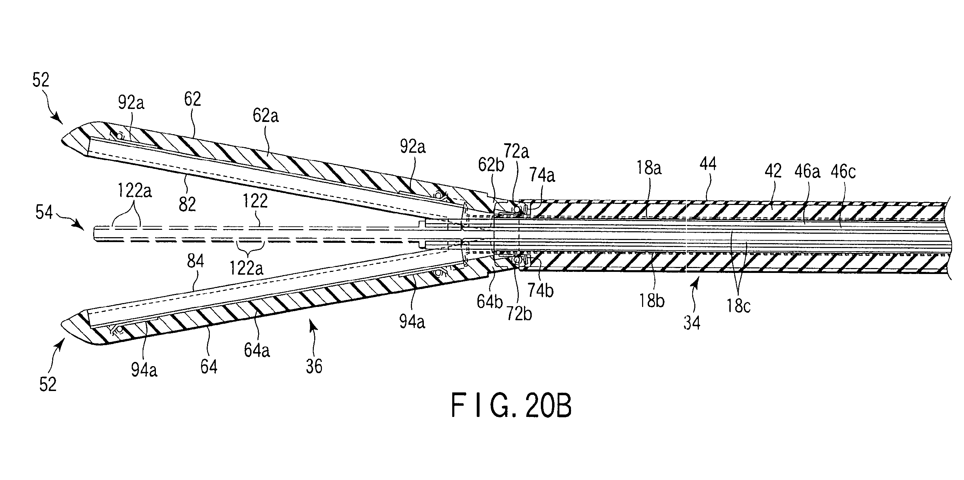

FIG. 20B is a schematic longitudinal sectional view showing the shaft and the treatment portion in which the first and second holding members are opened and in which the tubular electrode is disposed between the first and second holding members of the energy treatment instrument of the medical treatment apparatus according to the fifth embodiment;

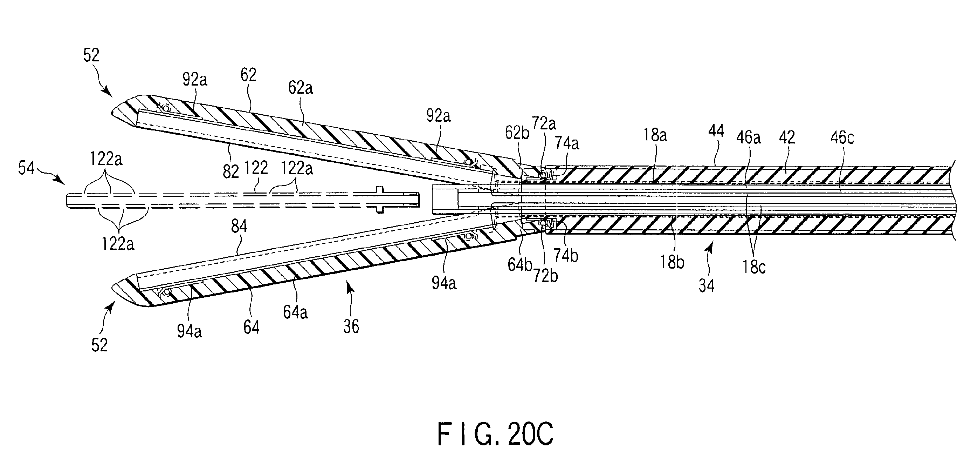

FIG. 20C is a schematic longitudinal sectional view showing the shaft and the treatment portion in which the first and second holding members are opened and in which the tubular electrode is separated from between the first and second holding members of the energy treatment instrument of the medical treatment apparatus according to the fifth embodiment;

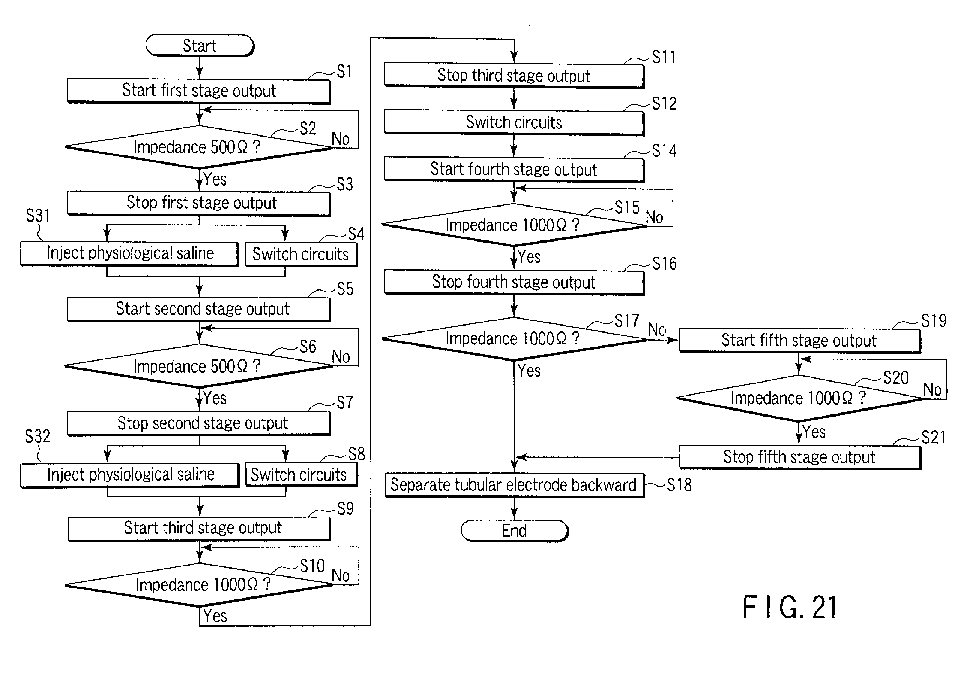

FIG. 21 a flowchart for joining living tissues by use of the medical treatment apparatus according to the fifth embodiment;

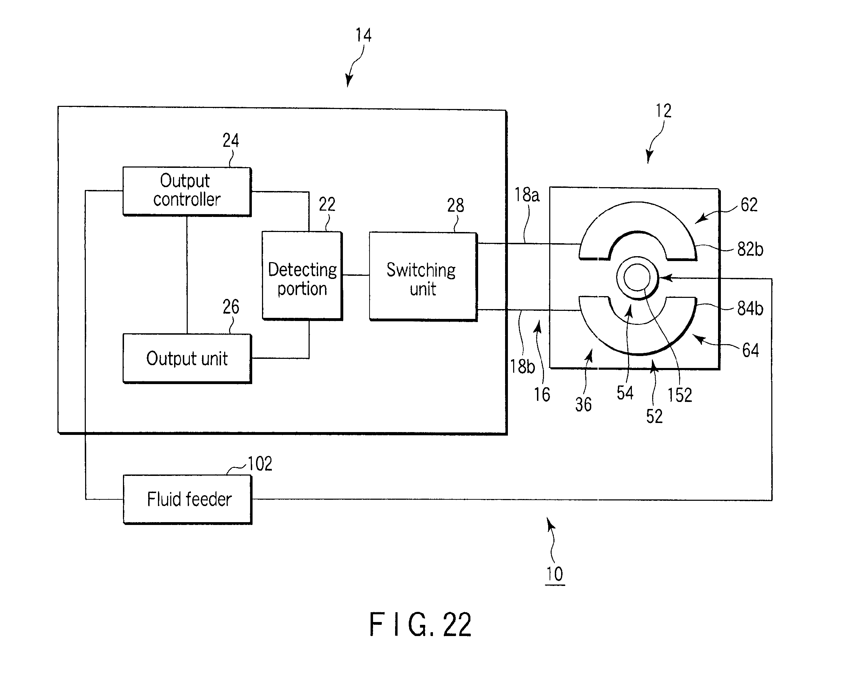

FIG. 22 is a schematic diagram showing a medical treatment apparatus according to a sixth embodiment;

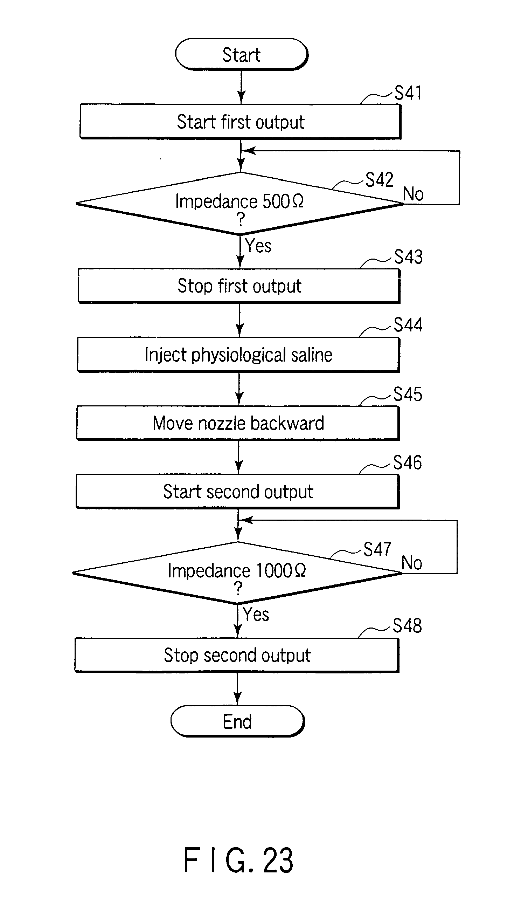

FIG. 23 a flowchart for joining living tissues by use of the medical treatment apparatus according to the sixth embodiment;

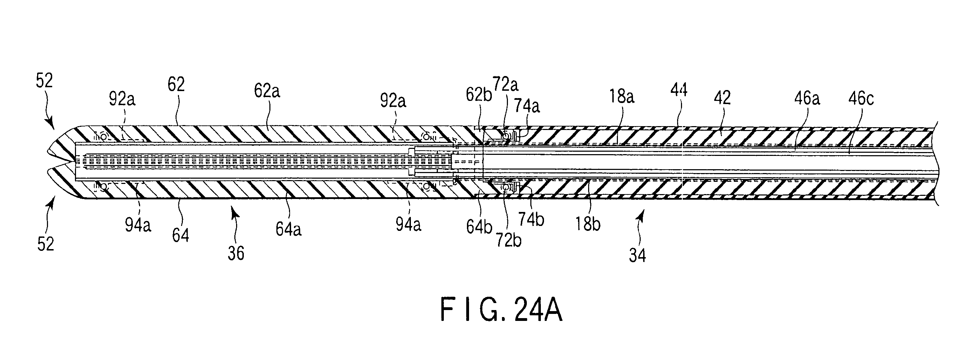

FIG. 24A is a schematic longitudinal sectional view showing a shaft and a treatment portion in which first and second holding members are closed and in which a liquid discharging nozzle is disposed between the first and second holding members of an energy treatment instrument in a medical treatment apparatus according to a seventh embodiment;

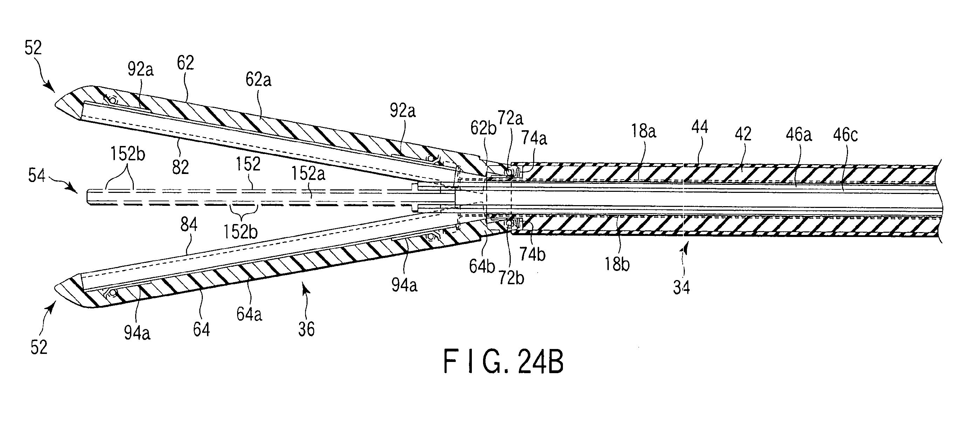

FIG. 24B is a schematic longitudinal sectional view showing the shaft and the treatment portion in which the first and second holding members are opened and in which the liquid discharging nozzle is disposed between the first and second holding members of the energy treatment instrument of the medical treatment apparatus according to the seventh embodiment; and

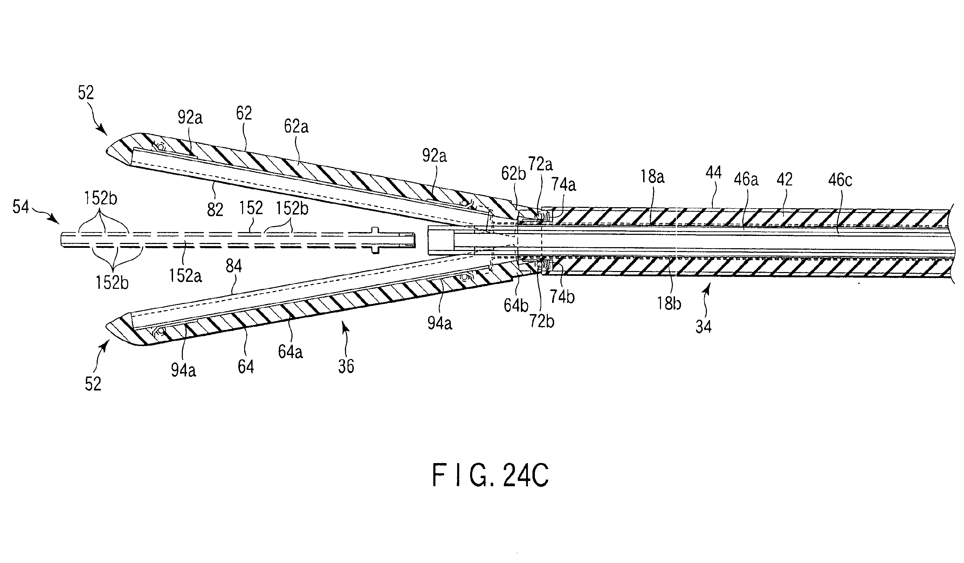

FIG. 24C is a schematic longitudinal sectional view showing the shaft and the treatment portion in which the first and second holding members are opened and in which the liquid discharging nozzle is separated from between the first and second holding members of the energy treatment instrument of the medical treatment apparatus according to the seventh embodiment.

### Best Mode for Carrying Out the Invention

The best mode for carrying out this invention will be described below with reference to the drawings.

### [First Embodiment]

A first embodiment is described with FIG. 1A to FIG. 10.

Here, a linear bipolar energy treatment instrument 12 for a treatment, for example, through an abdominal wall is described as an example of an energy treatment instrument.

As shown in FIG. 1A and FIG. 2, a medical treatment apparatus 10 includes an energy treatment instrument (medical treatment instrument) 12, and an energy source 14 for proving energy to a later-described treatment portion 36 of the energy treatment instrument 12. The energy treatment instrument 12 is detachably connected to the energy source 14 by a cable 16 extending from a later-described handle 32 and by a connector 16a provided at the end of the cable 16.

As shown in FIG. 2, the energy source 14 includes a detecting portion 22, an output controller 24, an output unit 26, and a switching unit 28. The detecting portion 22 is connected to the energy treatment instrument 12 via the switching unit 28. The output controller 24 and the output unit 26 are connected to the detecting portion 22, and the output controller 24 and the output unit 26 are connected together. The output unit 26 is connected to the energy treatment instrument 12 via the switching unit 28. Thus, the output unit 26 can supply energy to the energy treatment instrument 12 so that the output of the output unit is controlled by the output controller 24 at the same time.

In addition, the output unit 26 can pass a high-frequency current, and can also supply, for example, energy for heat generation and energy for an ultrasonic treatment.

The detecting portion 22 detects electric biological information obtained through a living tissue which is held (grasped) by later-described first and second holding members (a pair of holding members) 62, 64 of the energy treatment instrument 12 and which is in contact with later-described electrodes 82b, 84b. Here, the detecting portion 22 detects the values of a current and a voltage flowing through the living tissue held between the first and second holding members 62, 64, calculates the value of the impedance Z from the detected current and voltage values, and provides the calculated impedance Z as biological information. The output unit 26 outputs high-frequency energy under the control of the output controller 24. Thus, the output controller 24 can control the output of the high-frequency energy from the output unit 26 to the energy treatment instrument 12 on the basis of the biological information detected by the detecting portion 22.

In addition, when the high-frequency energy is applied to the electrodes holding the living tissue, the impedance Z, generally, once drops from about 50 [Ω] with time, and then rises again, as indicated in FIG. 3.

As shown in FIG. 1A, the energy treatment instrument 12 includes the handle (operation portion) 32, an elongate shaft 34 provided at the handle 32, and the treatment portion 36 provided at the distal end of the shaft 34.

The handle 32 is substantially L-shaped. The proximal end of the shaft 34 is fixed to one end of the handle 32. On the other hand, the other end of the handle 32 serves as a grip portion gripped by an operator (user of the energy treatment instrument 12).

The handle 32 is provided with a first knob (lengthwise feed lever) 32a side by side with the other end (grip portion) of the handle 32, and the first knob 32a serves to open and close the later-described first and second holding members 62, 64 of the treatment portion 36. If the first knob 32a is brought closer to or away from the other end of the handle 32, a later-described sheath 44 axially moves.

The handle 32 is provided, at its one end, with a second knob (lengthwise feed lever) 32b for moving a later-described rod electrode (third electrode) 66 of a second treating portion 54 along the axial direction of the shaft 34. As shown in FIG. 1B, the second knob 32b is connected, via a housing 43, to a lengthwise feed rod 46 which integrally has at its distal end the later-described rod electrode 66.

As shown in FIG. 4A to FIG. 4D, the shaft 34 includes a cylindrical member 42, and the sheath 44 slidably provided outside the cylindrical member 42. The cylindrical member 42 is fixed at its proximal end to the handle 32. The sheath 44 is slidable along the axial direction of the cylindrical member 42 by the operation of the first knob 32a. The lengthwise feed rod 46 which axially moves by the operation of the second knob 32b is provided inside the cylindrical member 42.

In addition, it is preferable that the outer peripheral surface of the lengthwise feed rod 46 and/or the inner peripheral surface of the cylindrical member 42 be insulated. For example, the outer peripheral surface of the lengthwise feed rod 46 is preferably covered with an insulating tube.

In this embodiment, the lengthwise feed rod 46 is integrally formed at the proximal end of the rod electrode 66 through the cylindrical member 42. Thus, when the second knob 32b of the handle 32 is moved to the distal side of the operator, the rod electrode 66 projects from the distal end of the cylindrical member 42 via the lengthwise feed rod 46 and is then located between the first and second holding members 62, 64, as shown in FIG. 4A and FIG. 4B. When the second knob 32b is moved toward the operator, the rod electrode 66 located between the first and second holding members 62, 64 axially moves toward the operator via the lengthwise feed rod 46, as shown in FIG. 4C. As a result, the distal end of the rod electrode 66 is stored in the distal end (treatment side end) of the cylindrical member 42.

As shown in FIG. 1A, the treatment portion 36 includes a first treating portion 52 and the second treating portion 54. The first treating portion 52 has the first and second holding members (a pair of holding members) 62, 64 which can open and close with respect to each other. The second treating portion 54 has the rod electrode 66 provided between the first and second holding members 62, 64.

In addition, the second holding member 64 has the same structure as the first holding member 62 shown in FIG. 5A and FIG. 5B, and therefore, the structure of the first holding member 62 is mainly described for representation. Moreover, the detailed structure of the second holding member 64 is not shown, but is properly provided with numerals for the purpose of explanation.

As shown in FIG. 1A, the first and second holding members 62, 64 are provided at the distal end of the shaft 34. The first holding member 62 integrally has a main body 62a and a base 62b. The second holding member 64 integrally has a main body 64a and a base 64b. In addition, the distal ends of the main bodies 62a, 64a of the first and second holding members 62, 64 are most distal to the handle 32, and the proximal ends of the main bodies 62a, 64a are most proximal to the handle 32. The first and second holding members 62, 64 have longitudinal axes determined by the distal ends and the proximal ends. Later-described grooves 92, 94 are formed along the longitudinal axes.

As shown in FIG. 4D, the outer surfaces of the main bodies 62a, 64a of the first and second holding members 62, 64 are smoothly curved. Although not shown, the outer surfaces of the bases 62b, 64b of the first and second holding members 62, 64 are also smoothly curved.

When the second holding member 64 is closed with respect to the first holding member 62, the cross sections of the main bodies 62a, 64a of the holding members 62, 64 are substantially circular or substantially elliptic as a whole. When the second holding member 64 is closed with respect to the first holding member 62, the cross sections of the bases 62b, 64b are substantially cylindrical as a whole. In this state, the diameters of the proximal ends of the main bodies 62a, 64a of the first and second holding members 62, 64 are greater than the diameters of the bases 62b, 64b. Thus, steps 63 are formed between the main bodies 62a, 64a and the bases 62b, 64b. The distal end of the sheath 44 of the shaft 34 comes into or out of contact with the steps 63 by the operation of the first knob 32a.

Here, when the second holding member 64 is closed with respect to the first holding member 62, the outer peripheral surfaces, which are substantially circular or substantially elliptic as a whole, of the bases 62b, 64b of the first and second holding members 62, 64 are substantially flush with or slightly greater in diameter than the outer peripheral surface of the distal end of the cylindrical member 42. Therefore, the sheath 44 of the shaft 34 can be slid over the cylindrical member 42 so that the bases 62b, 64b of the first and second holding members 62, 64 are covered with the distal end of the sheath 44.

The bases 62b, 64b of the first and second holding members 62, 64 are both supported rotatably around the distal end of the cylindrical member 42 of the shaft 34 in a direction perpendicular to the axial direction of the shaft 34 by support pins 72a, 72b which are disposed at the distal end of the cylindrical member 42. These support pins 72a, 72b are provided at the distal end of the cylindrical member 42 in parallel with each other. The bases 62b, 64b of the first and second holding members 62, 64 are rotated around the axes of the support pins 72a, 72b so that the main bodies 62a, 64a of the holding members 62, 64 can be opened or closed with respect to each other. The bases 62b, 64b of the first and second holding members 62, 64 are respectively urged by elastic members 74a, 74b such as leaf springs so that later-described holding surfaces 82, 84 of the main bodies 62a, 64a to be in contact with the living tissue are opened with respect to the position where the holding surfaces are in contact with each other. Actually, as shown in FIG. 4A to FIG. 4C, the elastic members 74a, 74b are provided on the outer peripheries of the support pins 72a, 72b provided at the distal end of the cylindrical member 42. Therefore, the bases 62b, 64b of the first and second holding members 62, 64 are urged in a direction to open.

Thus, when the first knob 32a is operated to move the distal end of the sheath 44 (forward) distally with respect to the operator, force is applied so that the bases 62b, 64b may be closed by the distal end of the sheath 44. Then, the first and second holding members 62, 64 close against the urging force by the elastic members 74a, 74b. In this case, if the living tissue is not in contact with the holding surfaces 82, 84 of the main bodies 62a, 64a of the first and second holding members 62, 64, the holding surfaces 82, 84 are in contact with each other. On the other hand, when the first knob 32a is operated to move the distal end of the sheath 44 (backward) proximally with respect to the operator, there is no force of the distal end of the sheath 44 to close the bases 62b, 64b, that is, the first holding member 62 and the second holding member 64 are opened due to the urging force by the elastic members 74a, 74b.

As shown in FIG. 5A, the first holding surface 82 for holding a treatment target living tissue is formed on the side of the main body 62a of the first holding member 62 proximate to the main body 64a of the second holding member 64. The second holding surface 84 for holding the treatment target living tissue is formed on the side of the main body 64a of the second holding member 64 proximate to the main body 62a of the first holding member 62. The first holding surface 82 has a first contact surface 82a which comes into contact with the living tissue when holding the living tissue, and a first electrode 82b as an energy output unit for outputting energy to the living tissue. The second holding surface 84 has a second contact surface 84a which comes into contact with the living tissue when holding the living tissue, and a second electrode 84b as an energy output unit for outputting energy to the living tissue.

As shown in FIG. 5A, the first and second contact surfaces 82a, 84a are flat. In addition, the distal ends of the first and second contact surfaces 82a, 84a are separate from each other even when the first and second holding members 62, 64 are closed. The first contact surface 82a is provided with the flat-plate-shaped first electrode 82b, as shown in FIG. 5B. The second contact surface 84a is provided with the flat-plate-shaped second electrode 84b. The first and second electrodes 82b, 84b are provided over the substantially entire surfaces of the first and second contact surfaces 82a, 84a except for their distal ends, as shown in FIG. 5A. In addition, the end face (side surface) of the first electrode 82b is aligned with the side surface of the main body 62a of the first holding member 62. The end face (side surface) of the second electrode 84b is aligned with the side surface of the main body 64a of the second holding member 64.

The first groove (recess) 92 where the rod electrode 66 is disposed is formed in the center of the contact surface 82a (first electrode 82b) of the holding surface 82 of the main body 62a of the first holding member 62. Similarly to the main body 62a, the second groove 94 is formed in the center of the contact surface 84a (second electrode 84b) of the holding surface 84 of the main body 64a of the second holding member 64 at a position opposite to the first groove 92 of the first holding member 62. The width of the grooves 92, 94 of the first and second main bodies 62a, 64a is greater than the width of the rod electrode 66. Moreover, the depth of the grooves 92, 94 of the first and second main bodies 62a, 64a is greater than half of the height of the rod electrode 66. Thus, when the first treating portion 52 is closed, that is, when the first and second holding members 62, 64 are closed, the rod electrode 66 is stored movably in and out without contacting the grooves 92, 94.

In addition, as shown in FIG. 5B, in order to apply sufficient high-frequency energy to the surface where the living tissue is removed, the groove 92 of the first holding member 62 is also provided with the first electrode (high-frequency electrode) 82b, and the groove 94 of the second main body 64a is also provided with the second electrode (high-frequency electrode) 84b. The first electrode 82b of the groove 92 of the first holding member 62 is formed to be discontinuous with but to have the same potential as the electrode 82b of the first contact surface 82a of the first holding member 62. Similarly, the second electrode 84b of the groove 94 of the second holding member 64 is formed to be discontinuous with but to have the same potential as the electrode 84b of the second contact surface 84a of the second holding member 64.

Elastic members 92a, 94a such as leaf springs (see FIG. 4A to FIG. 4C) are provided on the rear surfaces of the electrodes 82b, 84b disposed in the grooves 92, 94. The elastic members 92a, 94a can act together with the operation of the second knob 32b. When the rod electrode 66 is between the main bodies 62a, 64a of the first and second holding members 62, 64 as shown in FIG. 4A and FIG. 4B, the electrodes 82b, 84b disposed in the grooves 92, 94 are drawn in the main bodies 62a, 64a. When the rod electrode 66 is removed from between the main bodies 62a, 64a of the first and second holding members 62, 64 and thus drawn in the shaft 34 as shown in FIG. 4C, the electrodes 82b, 84b disposed in the grooves 92, 94 are pressed by the elastic members 92a, 94a and are flush with the holding surfaces 82, 84.

Thus, when the second knob 32b is disposed distally with respect to the operator, the elastic members 92a, 94a act together with the second knob 32b in such a manner as to draw the electrodes 82b, 84b disposed in the grooves 92, 94 into the main bodies 62a, 64a. On the other hand, when the second knob 32b is disposed proximally with respect to the operator, the elastic members 92a, 94a act together with the second knob 32b so that the electrodes 82b, 84b disposed in the grooves 92, 94 may be flush with the holding surfaces 82, 84 of the main bodies 62a, 64a. That is, the electrodes 82b, 84b disposed in the grooves 92, 94 are on the same surface as the electrodes 82b, 84b disposed on the holding surfaces 82, 84. Therefore, the elastic members 92a, 94a bring the electrodes 82b, 84b disposed in the grooves 92, 94 out of an urged state together with the forward movement of the rod electrode 66, and bring the electrodes 82b, 84b disposed in the grooves 92, 94 into an urged state together with the backward movement of the rod electrode 66. At the same time, the living tissue is also pressed by the electrodes 82b, 84b disposed in the grooves 92, 94.

The rod electrode 66 of the second treating portion 54 provided between the first and second holding members 62, 64 is the third electrode. The surface area of the rod electrode 66 is smaller than the addition of the surface areas of the first electrode 82b and the second electrode 84b of the first and second holding members 62, 64. Thus, the current density in the living tissue in contact with the surface of the rod electrode 66 is higher than the current density in the living tissue in contact with the surfaces of the first electrode 82b and the second electrode 84b. As a result, the living tissue around the rod electrode 66 (the living tissue in a deeper part) can be efficiently denatured.

As shown in FIG. 4D, the cross section of the rod electrode 66 is, for example, circular, but is permitted to have various shapes such as elliptic and polygonal shapes. The sectional shape of the grooves 92, 94 of the holding surfaces 82, 84 formed by the first and second holding members 62, 64 is preferred to be similar to that of the rod electrode 66, but is permitted to be various shapes such as circular, elliptic and polygonal shapes.

Disposed inside the cylindrical member 42 of the shaft 34 along its axial direction are a first conducting line 18a connected to the energy source 14 via the cable 16 extending from the handle 32, and a second conducting line 18b connected to the energy source 14 via the cable 16. One end of the first conducting line 18a is electrically connected to the first electrode 82b of the first holding member 62. The other end of the first conducting line 18a extends into the handle 32 through the cylindrical member 42 of the shaft 34. The first conducting line 18a is electrically connected to the connector 16a through the cable 16 extending from the handle 32. One end of the second conducting line 18b is electrically connected to the second electrode 84b of the second holding member 64. The other end of the second conducting line 18b extends into the handle 32 through the cylindrical member 42 of the shaft 34. The second conducting line 18b is electrically connected to the connector 16a through the cable 16 extending from the handle 32.

A third conducting line 18c is stored in the lengthwise feed rod 46 within the cylindrical member 42. Alternatively, one end of the third conducting line 18c is connected to the proximal end of the lengthwise feed rod 46 within the cylindrical member 42. The other end of the third conducting line 18c is connected to the connector 16a from within the handle 32 through the cable 16 extending from the handle 32.

Thus, energy can be supplied from the energy source 14 to the first electrode 82b, the second electrode 84b and the rod electrode 66 through the connector 16a and the first to third conducting lines 18a, 18b, 18c.

In addition, the first to third conducting lines 18a, 18b, 18c are electrically isolated from each other. The switching unit 28 of the energy source 14 can cause the first conducting line 18a to be homopolar (the same potential) or heteropolar (different potentials) with respect to the second conducting line 18b, and can cause the first and second conducting lines 18a, 18b to be heteropolar (different potentials) with respect to the third conducting line 18c. Thus, electricity is applied to the living tissue between the first electrode 82b and the rod electrode 66, to the living tissue between the second electrode 84b and the rod electrode 66, and to the living tissue between the first and second electrodes 82b, 84b, such that these living tissues can be denatured and dehydrated.

Furthermore, the first electrode 82b, the second electrode 84b and the rod electrode 66 can achieve a sensor function. That is, information on the living tissues can be transmitted to the energy source 14 from the first electrode 82b, the second electrode 84b and the rod electrode 66 through the first to third conducting lines 18a, 18b, 18c and the connector 16a.

As described above, the main body 64a of the second holding member 64 and the second electrode 84b are also formed symmetrically to the main body 62a of the first holding member 62 and the first electrode 82b. Thus, when the second holding member 64 is closed with respect to the first holding member 62, the high-frequency electrode 82b of the main body 62a of the first holding member 62 comes in contact with the high-frequency electrode 84b of the main body 64a of the second holding member 64 as shown in FIG. 4A. Then, when energy (high-frequency electric power) is supplied from the energy source 14, the living tissue in contact with the first high-frequency electrode 82b and the second high-frequency electrode 84b is supplied with the high-frequency electric power and heated.

At the same time, the first and second high-frequency electrodes 82b, 84b serve as sensors, and thus measure a current, voltage, etc. flowing the first and second high-frequency electrodes 82b, 84b through the living tissue, and then input relevant signals to the detecting portion 22 of the energy source 14 through the first and second conducting lines 18a, 18b.

Now, the effects of the medical treatment apparatus 10 according to this embodiment are described.

When an unshown power switch provided in the energy source 14 is, for example, pressed and turned on, the energy source 14 becomes operable (on standby).

As shown in FIG. 4A, the second holding member 64 is closed with respect to the first holding member 62, in which state the treatment portion 36 and the shaft 34 of the energy treatment instrument 12 is inserted into, for example, an abdominal cavity through an abdominal wall. The treatment portion 36 of the energy treatment instrument 12 is put face-to-face with the target living tissues (treatment target). At this point, the distal end of the rod electrode 66 may be inside or outside the cylindrical member 42 of the shaft 34.

The first knob 32a of the handle 32 is operated so that the target living tissues may be held (grasped) by the first holding member 62 and the second holding member 64. At the same time, the sheath 44 is moved with respect to the cylindrical member 42 toward the operator side of the shaft 34. Owing to the urging force by the elastic members 74a, 74b, a cylindrical space between the first and second bases 62b, 64b can not be maintained, and the first holding member 62 and the second holding member 64 open with respect to each other. Here, the first holding member 62 and the second holding member 64 simultaneously open at the same angle to the axial direction (central axis) of the shaft 34.

Then, the second knob 32b is operated to extend the rod electrode 66 with respect to the distal end of the cylindrical member 42 of the shaft 34. At the same time, one of the two treatment target living tissues (one living tissue) is disposed between the first high-frequency electrode 82b of the first holding member 62 and the rod electrode 66, and the other living tissue to be joined to the former living tissue is disposed between the second high-frequency electrode 84b of the second holding member 64 and the rod electrode 66. That is, the rod electrode 66 is disposed between the target living tissues so that the rod electrode 66 is held by the two living tissues, and the living tissues are disposed between the first and second holding members 62, 64.

In this state, the first knob 32a of the handle 32 is operated. At the same time, the sheath 44 is moved with respect to the cylindrical member 42 toward the distal side of the shaft 34. The space between the first and second bases 62b, 64b is closed and formed into a cylindrical shape by the sheath 44 against the urging force by the elastic members 74a, 74b. As a result, the main body 62a formed integrally with the base 62b of the first holding member 62 is closed with respect to the main body 64a formed integrally with the base 64b of the second holding member 64. Thus, the target two living tissues are held (grasped) between the first holding member 62 and the second holding member 64.

When tubular tissues or organs such as blood vessels or intestinal tracts are joined together, it is necessary to insert the rod electrode 66 into the tube such as blood vessels or intestinal tracts. It is also possible to insert the rod electrode 66 into the tube while applying electricity thereto. The rod electrode 66 also has a function of physical puncture with no application of energy. Therefore, the rod electrode 66 can be disposed on the joint surfaces of the tube after the first and second holding members 62, 64 are closed.

In this case, the target living tissues are in contact with both the first electrode 82b of the first holding member 62 and the second electrode 84b of the second holding member 64. Tissues around the target living tissues are in close contact with both the holding surface (contact surface, grasping surface) 82 of the first holding member 62 and the holding surface (contact surface, grasping surface) 84 of the second holding member 64 as well.

In this state, a foot switch or hand switch connected to the energy source 14 is operated. The effects of the medical treatment apparatus 10 are described below in detail along with a flowchart shown in FIG. 6.

Energy is output from the energy source 14 to living tissues between the first and second electrodes 82b, 84b and the rod electrode 66 via the first to third conducting lines 18a, 18b, 18c provided in the cable 16 (S1).

In this case, a circuit is switched and set by the switching unit 28 so that a current may be passed to the living tissues between the rod electrode 66 and the first and second electrodes 82b, 84b. The circuit set by the switching unit 28 in this manner is referred to as a circuit of a first stage output. At the same time, the rod electrode 66 has a polarity different from the polarity of the first and second electrodes 82b, 84b. On the other hand, the first and second electrodes 82b, 84b are homopolar. Such an output state is referred to as the first stage output. The first stage output is intended for a bipolar treatment wherein electricity is applied to the living tissues between the rod electrode 66 and the first and second electrodes 82b, 84b. In addition, in the first stage output, in order to avoid complete dehydration, the threshold value (stop impedance) of the impedance Z is set to an impedance (500 [Ω] as described later) lower than a high impedance of, for example, 1000 [Ω] or more (see FIG. 3) at which complete dehydration is achieved.

The treatment of the living tissues by high-frequency energy (here, a treatment of joining two living tissues together) is started, and at the same time, the impedance Z of the living tissues in contact with the rod electrode 66 is detected by the detecting portion 22 which receives a signal within the energy source 14 from the rod electrode 66. As shown in FIG. 3, the impedance Z at the beginning of the treatment (initial value) is, for example, about 50 [Ω], which however varies depending on the size (contact area) and shape of the electrodes 82b, 84b. Then, as high-frequency electric power is applied to the living tissues and the living tissues are cauterized, the value of the impedance Z once drops from about 50 [Ω], and then rises. Such a rise in the value of the impedance Z represents that the living tissues are losing water and drying (dehydrated).

That is, in the first stage output, a high-frequency current is applied to the first and second electrodes 82b, 84b of the first and second holding members 62, 64 and the rod electrode 66. As a result, the living tissues in contact with the electrodes 82b, 84b generate heat. That is, Joule heat is generated within the living tissues grasped between the electrodes 82b, 84b so that the living tissues themselves are heated. The high-frequency energy denatures proteins contained in the living tissues. At the same time, the living tissues themselves generate heat and are dehydrated. Consequently, proteins bond with each other, such that components constituting the living tissues bond with each other.

In this case, the contact area between the first and second electrodes 82b, 84b and the living tissues is greater than the contact area between the rod electrode 66 and the living tissues. In other words, the contact area between the rod electrode 66 and the living tissues is smaller than the contact area between the first and second electrodes 82b, 84b and the living tissues. Therefore, the current density in the living tissue in contact with the rod electrode 66 is higher than the current density in the living tissue in contact with the first and second electrodes 82b, 84b. As a result, tissue components in the surface of the living tissue in contact with the rod electrode 66 are denatured more than tissue components in the surface of the living tissue in contact with the first and second electrodes 82b, 84b. Thus, the living tissues can be denatured particularly in the joint surfaces. In this manner, denatured region of the living tissues can be gradually expanded from the parts in contact with the rod electrode 66 to deeper parts of the living tissues. Thus, joining strength in the joint surfaces of the living tissues gradually increase.

Then, it is judged whether the calculated impedance Z has exceeded, for example, 500 [Ω] (not limited to this value and any value can be set) set as the threshold value in the output controller 24 (S2). In this case, the impedance Z is set so that water contained in the target living tissues may not be completely removed to allow for the denaturation of the living tissues.

When the impedance Z is judged to have exceeded a threshold value of 500 [Ω] in accordance with the detection by the detecting portion 22, the output controller 24 stops the output of the high-frequency electric power from the output unit 26 (S3).

When the output of the high-frequency electric power from the output unit 26 is stopped by the output controller 24, the switching unit 28 simultaneously switches the conducting lines 18a, 18b, 18c which connect the output controller 24, the energy treatment instrument 12 and the detecting portion 22 (S4). In this case, the conducting lines are switched so that electricity is applied to the living tissues between the first electrode 82b and the second electrode 84b of the first and second holding members 62, 64. No energy is supplied to the rod electrode 66 connected to the third conducting line 18c. The circuit set by the switching unit 28 in this manner is referred to as a circuit of a second stage output. Such an output state is referred to as the second stage output. The second stage output is intended for a bipolar treatment wherein electricity is applied to the living tissues between the first and second electrodes 82b, 84b.

When the foot switch or hand switch is operated, the second stage output is started (S5). Simultaneously with the start of the output, the impedance Z of the living tissues is detected as in first stage output. Whether the impedance has reached, for example, 500 [Ω] is judged (S6). When the impedance has reached 500 [Ω], the output is stopped (S7). In this output as well, a lower impedance of, for example, 500 [Ω] is set as a threshold value as described above so that water contained in the tissues may not be completely removed.

That is, in the second stage output, electricity is applied to the living tissues between the electrode 82b of the first holding member 62 and the electrode 84b of the second holding member 64. It is therefore possible to evenly denature the living tissues between the first and second electrodes 82b, 84b including the joint surfaces thereof.

Then, the switching unit 28 is switched to the same circuit as the circuit of the first stage output (S8). Electricity is applied to the living tissues between the rod electrode 66 and the first and second electrodes 82b, 84b (S9). Then, the joint surfaces of the tissues are completely dehydrated by an output with an impedance of, for example, 1000 [Ω] as a stop condition (threshold value) (S10). Such an output state is referred to as a third stage output. The third stage output is intended for a bipolar treatment wherein electricity is applied to the living tissues between the rod electrode 66 and the first and second electrodes 82b, 84b. That is, whether the impedance Z detected by the detecting portion 22 reaches 1000 [Ω] is judged, and the output is stopped when the impedance has reached 1000 [Ω] (S11).

That is, in the third stage output, electricity is applied to the living tissues between the rod electrode 66 and the first and second electrodes 82b, 84b, so that the living tissues around the rod electrode 66 with a high current density can be completely dehydrated.

Then, the switching unit 28 is switched to the circuit of the second stage output (S12).

Simultaneously with, before or after the switch (S12) of the switching unit 28 to the circuit of the second stage output, the rod electrode 66 between the first and second holding members 62, 64 is moved backward (S13). If the second knob 32b provided in the handle 32 is moved toward the operator, the rod electrode 66 disposed between the first and second holding members 62, 64 of the energy treatment instrument 12 axially moves toward the operator through the cylindrical member 42 of the shaft 34, and the distal end of the rod electrode 66 is stored in the end (distal end) of the cylindrical member 42 of the shaft 34 on the side of the treatment portion 36.

When the second knob 32b is thus operated to move the rod electrode 66 backward to the side proximate to the operator, the electrodes 82b, 84b disposed in the grooves 92, 94 move toward the holding surfaces 82, 84 due to the urging force by the elastic members 92a, 94a. Thus, the electrodes 82b, 84b disposed in the grooves 92, 94 apply pressure to the living tissues.

As a result, pressure can be applied to the living tissues by the entire holding surfaces 82, 84 of the first and second holding members 62, 64 so that the space where the rod electrode 66 has been disposed may be closed. Thus, the joint surfaces of the target living tissues can be brought to a joined state (contact state).

As described above, since the switching unit 28 is switched to the circuit of the second stage output, the foot switch or hand switch is operated to perform a fourth stage output (S14). The fourth stage output is intended for a bipolar treatment wherein electricity is applied to the living tissues between the first and second electrodes 82b, 84b. The fourth stage output is performed with a stop condition (threshold value) of, for example, 1000 [Ω] (S15). Electricity is applied to the living tissues between the electrodes 82b, 84b of the first and second holding members 62, 64 in order to denature and dehydrate the held living tissues. Further, the impedance Z is detected by the detecting portion 22 simultaneously with the output from the output unit 26, and the output is stopped when the impedance Z is 1000 [Ω] (S16).

In the fourth stage output, electricity is applied to the living tissues between the electrodes 82b, 84b of the first and second holding members 62, 64, so that water in the parts of the living tissues separated by the disposed rod electrode 66 is completely removed, that is, the living tissues can be dehydrated and also joined together. Subsequently, the treatment can be stopped.

The treatment described above makes it possible to heat the target living tissues and gradually denature and dehydrate and thus unite (join, fuse) the living tissues. As described above, the circuits are switched by the switching unit 28, and high-frequency energy is output to the living tissues. This ensures that energy can be input to the joint surfaces of the target living tissues that are thick or different in composition. It is therefore possible to ensure the denaturation and dehydration of the target living tissues.

In addition, the start and stopping of the output of each stage is preferably displayed in the energy source 14 or sounded (e.g., buzzed) so that the operator may recognize.

It is also advantageous to provide an idle period of, for example, several seconds in the high-frequency output or to repeat lower outputs and high outputs.

As to a termination condition (stop condition), the output of each stage may be automatically ended not only judging whether the impedance Z has exceeded the threshold value set as the termination condition but also after the high-frequency energy is output for a certain period of time.

Furthermore, although the application of electricity to the rod electrode 66, the backward movement of the rod electrode 66 and the output of energy to the living tissues between the first and second holding members 62, 64 are manually performed here, the series of operations may be set to be automatically performed. In this case, the series of operations (the application of electricity to the rod electrode 66, the backward movement of the rod electrode 66 and the output of energy to the living tissues between the holding members 62, 64) are preferably automatically started and automatically ended, for example, by keeping the foot switch or hand switch pressed by the operator. Moreover, the series of operations is preferably set to be, when being performed, forcibly ended by releasing the foot switch or hand switch. In addition, the rod electrode 66 is provided with an unshown motor connected to the lengthwise feed rod 46, and the motor is controlled by the output controller 24 so that the rod electrode 66 may be automatically moved backward in accordance with a series of effects.

Moreover, in the operation described above, the first and second electrodes 82b, 84b are homopolar, so that output is provided to the living tissues between the rod electrode 66 and the first and second electrodes 82b, 84b of the first and second holding members 62, 64 and the biological information obtained therefrom is detected. Otherwise, output and the detection of the biological information may be performed using only one of the electrodes 82b, 84b of the first and second holding members 62, 64; for example, only the living tissue between the rod electrode 66 and the electrode 82b of the first holding member 62 or only the living tissue between the rod electrode 66 and the electrode 84b of the second holding member 64. When the living tissues different in thickness are joined together, for example, when the esophagus and the small intestine are joined together, highly precise control can be achieved if output and the detection of the biological information are performed using only one of the electrodes 82b, 84b of the first and second holding members 62, 64. It goes without saying that the use of both the electrodes 82b, 84b is also suitable. The circuits can be switched by the switching unit 28 in this case as well.

As described above, the following can be said according to this embodiment.

The living tissues are joined together by the denaturation of proteins and dehydration. That is, in order to fuse (join) the living tissues together by energy, it is important to denature the proteins of the living tissues and to remove water contained in the living tissues (dehydration). The denaturation of proteins and dehydration are achieved by causing the living tissues to generate heat. A living body has tissues constituted of various proteins. Denaturation temperature varies protein by protein, so that ensuring a temperature rise in the living tissues is necessary to join the living tissues together independently of the composition of proteins.

When the thickness of the target living tissues held by the holding members 62, 64 is small, energy can be substantially uniformly applied to the held living tissues. However, when the target living tissues held by the holding members 62, 64 are thick, the density of energy provided to the living tissues is low and sufficient energy may not be applied in the joint surfaces (deeper portions) of the held living tissues farthest from the electrodes 82b, 84b.

In the present embodiment, the rod electrode 66 is disposed directly on the joint surfaces of the target living tissues so that electricity can be applied to the living tissues between the electrodes 82b, 84b of the first and second holding members 62, 64. Therefore, high-frequency energy can concentrate on (energy density can be increased) the joint surfaces where the rod electrode 66 is disposed. This ensures that more energy can be applied to the joint surfaces of the living tissues, and proteins in the joint surfaces of the living tissues can be denatured equally to or more than in other parts. Dehydration can also be achieved equally to or more than in other parts. That is, control of the treatment state in the joint surfaces can be ensured. Thus, proteins of the living tissues located away from the electrodes 82b, 84b provided in the holding surfaces 82, 84 of the pair of holding members 62, 64 (e.g., located on the joint surfaces of the living tissues) can be sufficiently denatured. The part of the living tissues located far (deep) from the electrodes 82b, 84b of the holding members 62, 64 can also be sufficiently dehydrated. That is, the denaturation state and dehydration state of proteins in the deep parts can be equal independently of the thickness and composition of the living tissues. Consequently, the living tissues can be joined together more stably and treated more safely than in the case of conventional techniques.

According to the present embodiment, the combination of the electrodes 82b, 84b, 66 for the treatment are switched while the state of the target living tissues held by the first and second holding members 62, 64 is being monitored by the impedance Z using the electrodes 82b, 84b, 66. Thus, energy efficiently concentrates on the joint surfaces of the living tissues, thereby further ensuring that the joint surfaces of the living tissues can be denatured and dehydrated.

In addition, although the living tissues are joined together in the example described here, the living tissues can also be simply held and coagulated.

Furthermore, when it is difficult to move the rod electrode 66 forward and backward, it is also preferable to rotate the rod electrode 66 around its axis. For example, a motor (included in the numeral 43 in FIG. 1B) is provided in the housing 43 which connects the second knob 32b to the lengthwise feed rod 46, such that the rod electrode 66 can be rotated together with the lengthwise feed rod 46. When the rod electrode 66 is thus rotatable, it is possible to more easily draw the rod electrode 66 from the joint surfaces of the living tissues or insert the rod electrode 66 into the joint surfaces of the living tissues by combining with the backward and forward movement of the rod electrode 66.

The medical treatment apparatus 10 has been described with FIG. 1A to FIG. 6 in the present embodiment, but is not limited to the parts having the configuration described above. The parts can be replaced with any parts having similar functions. Although not shown, similar effects can be obtained if the high-frequency electrodes 82b, 84b of the holding members 62, 64 are replaced with, for example, heater elements which output heat energy from resistance heating. Moreover, similar effects can also be obtained if the rod electrode 66 is replaced with, for example, a heater element or ultrasonic vibration. The output unit 26 of the energy source 14 is preferably capable of outputting all of high-frequency energy, heat energy and ultrasonic energy.

Although the status (state) of the living tissues is detected, that is, monitored by the impedance Z in the present embodiment, the biological information is not limited to the impedance Z. For example, the use of other electric information such as an electric power value or a phase is also permitted. That is, the biological information includes, for example, a current, a voltage and electric power for calculating the impedance Z, the impedance Z calculated therefrom, and phase information.

It is also preferable that the electrodes 82b, 84b of the holding members 62, 64 be formed and arranged as shown in FIG. 7A and FIG. 7B. In this case, the electrodes 82b, 84b are circular. Moreover, some of the electrodes 82b disposed in the groove 92 may be arranged proximately to each other instead of the arrangement at equal intervals. When the electrodes 82b are thus arranged, it is possible to perform a treatment wherein current density is increased for the opposite electrode 84b of the holding member 64.

Moreover, in order to sufficiently treat the surfaces of the living tissues held by the holding surfaces 82, 84 of the main bodies 62a, 64a of the first and second holding members 62, 64, it is also preferable that the electrodes 82b, 84b be continuously formed in a direction perpendicular to the longitudinal direction of the main bodies 62a, 64a, as shown in FIG. 8A to FIG. 8D (FIG. 8D in particular). The use of such electrodes 82b, 84b makes it possible to have a wider contact area between the electrodes 82b, 84b and the living tissues and to apply pressure sufficient to treat the living tissues without disposing the elastic members 92a, 94a (see FIG. 4A to FIG. 4C).

Furthermore, in the case described in this embodiment, the second knob 32b separate from the first knob 32a is used to move the lengthwise feed rod 46 and the rod electrode 66 with respect to the shaft 34, as shown in FIG. 1A and FIG. 1B. Otherwise, it is also preferable that the first knob 32a and the second knob 32b be provided side by side, as shown in FIG. 9. When the second knob 32b shown in FIG. 9 is brought closer to the other end of the handle 32, the distal end of the rod electrode 66 is retracted into the distal end of the shaft 34. When the second knob 32b is brought away from the other end of the handle 32, the distal end of the rod electrode 66 projects from the distal end of the shaft 34 and is located between the first and second holding members 62, 64.

Still further, the linear energy treatment instrument 12 (see FIG. 1A) for treating living tissues in an abdominal cavity (in the body) through an abdominal wall has been described as an example in this embodiment. However, it is also possible to use, for example, open linear energy treatment instrument (treatment instrument) 12a shown in FIG. 10 for taking target living tissues out of the body through an abdominal wall and then treating the same. The energy treatment instrument 12a includes a handle 32 and a treatment portion 36. That is, the energy treatment instrument 12a has no shaft 34 (see FIG. 1A) in contrast with the energy treatment instrument 12 for treating through an abdominal wall. On the other hand, a member having the same function as the shaft 34 is provided in the handle 32 of the energy treatment instrument 12a. Thus, the energy treatment instrument 12a can be used similarly to the above-described energy treatment instrument 12 shown in FIG. 1A.

Although not shown, a plurality of electrodes such as fourth and fifth electrodes provided side by side with the rod electrode (third electrode) 66 are also allowed to be disposed between the first and second holding members 62, 64, in addition to the rod electrode (third electrode) 66 shown in FIG. 1A,

FIG. 2, FIG. 4A to FIG. 4D. The fourth and fifth electrodes referred to here are arranged, for example, with the rod electrode 66 interposed in between.

### [Second Embodiment]

Next, a second embodiment is described with FIG. 11 to FIG. 14. This embodiment is a modification of the first embodiment, and the same parts as the parts described in the first embodiment are provided with the same numerals and are not described in detail.

In the present embodiment, for a rod electrode 66 present between first and second holding members 62, 64, there is disposed a mechanism capable of separating the rod electrode 66, in addition to a mechanism capable of moving along the axial direction of a shaft 34.

As shown in FIG. 11, a second knob 32b of a handle 32 is provided with a separation knob 32c for separating the rod electrode 66 at the distal end of a later-described lengthwise feed tube 46a. The proximal end of a later-described connection rod 46b is connected to the second knob 32b, and the proximal end of the lengthwise feed tube 46a is connected to the separation knob 32c. The separation knob 32c generally moves together with the second knob 32b. Here, the separation knob 32c is provided on the distal side of the operator with respect to the second knob 32b.

As shown in FIG. 12A to FIG. 12C, the cylindrical lengthwise feed tube 46a and the connection rod 46b are provided instead of the lengthwise feed rod 46 inside a cylindrical member 42 of the shaft 34. The connection rod 46b is provided inside the cylindrical lengthwise feed tube 46a. Further, a cylindrical concave portion (depression) 66a is formed at the proximal end of the rod electrode 66, and the distal end of the connection rod 46b is fitted into the concave portion 66a. Moreover, the proximal end of the rod electrode 66 is in contact with the distal end of the cylindrical lengthwise feed tube 46a.

The connection rod 46b is formed of a conductive material. A third conducting line 18c connected to an output unit 26 of an energy source 14 is connected to the end of the connection rod 46b on the side of the handle 32. The lengthwise feed tube 46a is preferably formed of an insulating material such as rigid plastic. Thus, electricity can be applied to electrodes 82b, 84b of the first and second holding members 62, 64 from the rod electrode 66 through a living tissue.

The distal end of the connection rod 46b is fitted into the concave portion 66a at the proximal end of the rod electrode 66 to the extent that the fit is not undone when the rod electrode 66 is moved (including pushing/drawing of the rod electrode 66 held by the living tissue) as described in the first embodiment.

On the other hand, if the separation knob 32c is separated from the second knob 32b located on the distal side of the operator and is thus moved to the distal side of the operator, the connection rod 46b remains where it is, but the lengthwise feed tube 46a moves to the distal side of the operator. As a result, the proximal end of the rod electrode 66 is pushed by the distal end of the lengthwise feed tube 46a, thus undoing the fitting of the distal end of the connection rod 46b in the concave portion 66a at the proximal end of the rod electrode 66. That is, the rod electrode 66 is separated from the lengthwise feed tube 46a and the connection rod 46b.

Alternatively, the lengthwise feed tube 46a remains where it is instead of the connection rod 46b, and the connection rod 46b is moved toward the operator relative to the lengthwise feed tube 46a, such that the rod electrode 66 is also separated from the lengthwise feed tube 46a and the connection rod 46b. That is, the separation knob 32c remains where it is, and the second knob 32b is moved toward the operator relative to the separation knob 32c, such that the rod electrode 66 is also separated from the lengthwise feed tube 46a and the connection rod 46b.

Moreover, after the separation of the rod electrode 66, a new rod electrode 66 is fitted to the connection rod 46b if necessary, so that an energy treatment instrument 12 having the rod electrode 66 can be used.

As in the first embodiment, elastic members 92a, 94a are disposed within the holding members 62, 64, as shown in FIG. 12A to FIG. 12C. The elastic members 92a, 94a bring the electrodes 82b, 84b disposed in grooves 92, 94 into an urged state along with the operation of locating the second knob 32b and the separation knob 32c proximally to the operator (the operation of moving the rod electrode 66 backward), while the elastic members 92a, 94a bring the electrodes 82b, 84b disposed in the grooves 92, 94 out of the urged state along with the operation of locating the second knob 32b and the separation knob 32c distally to the operator (the operation of moving the rod electrode 66 forward) (see FIG. 4A to FIG. 4C).

Then, as shown in FIG. 12C, the second knob 32b and the separation knob 32c are located distally to the operator even when the rod electrode 66 is separated, the urging of the electrodes 82b, 84b disposed in the grooves 92, 94 is undone. Here, in order to bring the electrodes 82b, 84b disposed in the grooves 92, 94 into an urged state, it is only necessary to locate the second knob 32b and the separation knob 32c proximally to the operator.

The effects of a medical treatment apparatus 10 are described below along with a flowchart shown in FIG. 13.

Here, the step of moving the rod electrode 66 backward (S13) in the flowchart (see FIG. 6) in the first embodiment is eliminated, and the step of separating the rod electrode (probe) 66 (S18) is inserted instead. The step of separating the rod electrode 66 (S18) is performed after a fourth stage output is stopped.

Living tissues are held as described in the first embodiment, and the steps from the start of a first stage output (S1) to the stopping of the fourth stage output (S16) are carried out. Then, the impedance Z of the living tissues between the electrodes 82b, 84b of the first and second holding members 62, 64 and the rod electrode 66 is detected (S17).

If the detected value of the impedance Z is dropped from the value at which the fourth stage output is stopped (S16), the living tissues between the electrodes 82b, 84b of the first and second holding members 62, 64 and the rod electrode 66 is again treated by the application of electricity as a fifth stage output (S19). The fifth stage output is performed with an impedance of, for example, 1000 [Ω] as a stop condition (threshold value) (S20). Such an output state is referred to as the fifth stage output. That is, whether the impedance Z detected by a detecting portion 22 reaches 1000 [Ω] is judged, and the output is stopped when the impedance has reached 1000 [Ω] (S21). The fifth stage output enables more complete dehydration of the held living tissues. This is carried out due to the possibility of a phenomenon in which water returns into the living tissues during the fourth stage output or after the fourth stage output has been stopped.

In this case, if electricity is applied to the living tissues between the electrodes 82b, 84b of the first and second holding members 62, 64 by the fourth stage output or the fifth stage output, the joint surfaces of the living tissues including the living tissues in contact with the rod electrode 66 are denatured and dehydrated.

After the fourth stage output or the fifth stage output is stopped, the rod electrode 66 provided between the holding members 62, 64 is separated. As a result, the rod electrode 66 is separated. That is, the rod electrode 66 remains placed on the joint surfaces of the living tissues.

Even when the rod electrode 66 remains placed on the joint surfaces of the living tissues as mentioned above, the joint surfaces can be preserved because the joint surfaces of the living tissues including the living tissues in contact with the rod electrode 66 have been denatured and dehydrated. Moreover, it is also possible to prevent the joint surfaces from being peeled off due to the removal of the rod electrode 66 from the joint surfaces of the living tissues.

As described above, the following can be said according to this embodiment.

There is a possibility of the phenomenon in which water returns into the living tissues during the fourth stage output or after the fourth stage output has been stopped. Therefore, in the present embodiment, the impedance Z of the joint surfaces of the living tissues is again detected after the fourth stage output has been stopped, and if necessary, the fifth stage output is performed for the joint surfaces of the living tissues. This enables more complete dehydration of the living tissues. Moreover, since the rod electrode 66 remains placed on the joint surfaces of the living tissues, there is no need to consider the removal of the rod electrode 66. Therefore, pressure can be applied to the joint surfaces of the living tissues not only by holding surfaces 82, 84 of the first and second holding members 62, 64 but also by the electrodes 82b, 84b disposed in the grooves 92, 94. This enables the living tissues to be more firmly joined together.

In addition, in order to sufficiently treat the surfaces of the living tissues held by the first and second holding members 62, 64, it is also preferable that the electrodes 82b, 84b be continuously formed in a direction perpendicular to the longitudinal direction of main bodies 62a, 64a, as shown in FIG. 14A to FIG. 14D (FIG. 14D in particular). The use of such electrodes 82b, 84b makes it possible to apply pressure sufficient to treat the living tissues without disposing the elastic members 92a, 94a (see FIG. 12A to FIG. 12C) in the holding members 62, 64 shown in FIG. 14A to FIG. 14D.

### [Third Embodiment]

A third embodiment is described with FIG. 15 to FIG. 18. This embodiment is a modification of the first embodiment, and the same parts as the parts described in the first embodiment are provided with the same numerals and are not described in detail.

As shown in FIG. 15 and FIG. 16, a medical treatment apparatus 10 includes a fluid feeding device 102 for feeding a treatment portion 36 with a fluid such as a physiological saline, in addition to an energy treatment instrument (handpiece, treatment instrument) 12, and an energy source 14 for sending high-frequency energy to the energy treatment instrument 12.

The fluid feeding device 102 includes a bag 112 containing the physiological saline, a conveying tube (fluid feeding tube) 114 to which the rear end of the bag 112 is connected, and a flow volume adjuster (fluid controller) 116 for changing the inside diameter of the conveying tube 114 to control the volume of the physiological saline fed from the bag 112.

As the fluid retained in the bag 112, it is preferable to use a fluid capable of inducing electric energy, such as an ionized conductive fluid permeable to living tissues. Such a fluid used includes, for example, a physiological saline, a hypertonic saline, a hypotonic saline or an electrolyte fluid replacement drug. The use of a highly viscous gel (fluid) such as hyaluronic acid is also permitted. In the case where the gel is used, the gel permeates into a treatment target living tissue when applied thereto, but the gel is prevented from flowing to living tissues around the treatment target living tissue.

The conveying tube 114 extends from a handle 32 of the energy treatment instrument 12 similarly to a cable 16. The flow volume adjuster 116 is detachably connected to the energy source 14 by a cable 118 and a connector 118a provided at its end. When the feeding of the physiological saline from the bag 112 is stopped, the flow volume adjuster 116, for example, externally pressures the conveying tube 114 to block the flow path of the conveying tube 114. When the physiological saline is fed from the bag 112, the flow volume adjuster 116, for example, removes the external pressure on the conveying tube 114 to open the flow path of the conveying tube 114 by the elastic deformation of the conveying tube 114. That is, the flow volume adjuster 116 can mechanically open and close the flow path of the conveying tube 114.

An unshown foot switch or hand switch for opening and closing the flow path of the conveying tube 114 is connected to the flow volume adjuster 116. For example, when the foot switch or hand switch is kept unpressed, the feeding of the physiological saline to the energy treatment instrument 12 is stopped. On the other hand, when the foot switch or hand switch is kept pressed, a controlled flow volume of physiological saline is fed to the energy treatment instrument 12 through the conveying tube 114, and the flow path of the conveying tube 114 is automatically blocked so that the feeding of the physiological saline is automatically stopped. Here, various controlled flow volume can be set; for example, to allow a predetermined flow volume, to allow the fluid (physiological saline) to flow for a predetermined period of time, or to allow the fluid (physiological saline) to be fed suitably to an output such as a second stage output.

In addition, a flow volume adjusting knob 32d is provided side by side with a first knob 32a in this embodiment. The flow volume adjusting knob 32d is used to forcibly open or close the flow volume adjuster 116.

The distal end of the conveying tube 114 is connected to the proximal end of a lengthwise feed tube 46a shown in FIG. 17A to FIG. 17C. The rear end of the lengthwise feed tube 46a is connected to a second knob 32b of the handle 32. Thus, the lengthwise feed tube 46a moves backward and forward through a cylindrical member 42 of a shaft 34 by the operation of the second knob 32b.

The proximal end of a tubular electrode 122 having a flow path 122a is connected in a fitted state to the distal end of the lengthwise feed tube 46a. The distal end of the third conducting line 18c is connected to the proximal end of the tubular electrode 122. The third conducting line 18c extends to the side of the handle 32 through the lengthwise feed tube 46a, and extends in the handle 32 from the lengthwise feed tube 46a to the outside, and then disposed in the cable 16 together with first and second conducting lines 18a, 18b.

That is, the tubular electrode 122 according to this embodiment is a cylindrical form of the rod electrode (high-frequency electrode 66) described in the first and second embodiments. Thus, the tubular electrode 122 has the function of discharging a liquid to a living tissue which may be present between first and second holding members 62, 64.

As shown in FIG. 15, the handle 32 is provided with the second knob (liquid discharger equipped knob for electrode forward/backward movement) 32b. As shown in FIG. 17A to FIG. 17C, the second knob 32b is provided with the lengthwise feed tube 46a instead of the lengthwise feed rod 46. The distal end of the lengthwise feed tube 46a is connected to the proximal end of the tubular electrode 122 through the cylindrical member 42 of the shaft 34. The third conducting line 18c is inserted through the lengthwise feed tube 46a.

As shown in FIG. 15 to FIG. 17D, the tubular electrode 122 is provided between the first and second holding members 62, 64. The tubular electrode 122 can be moved forward and backward between a condition to be located between the first and second holding members 62, 64 (the tubular electrode 122 projects) and a condition to be disposed within the shaft 34. The distal end of the tubular electrode 122 when projected is set at the position which is not beyond the most distal electrodes 82b, 84b among the electrodes 82b, 84b of the first and second holding members 62, 64. Moreover, the width (outside diameter) of the tubular electrode 122 is smaller than the electrodes 82b, 84b of the first and second holding members 62, 64. Thus, energy application can be limited to the surfaces of the living tissues to which energy is applied by the electrodes 82b, 84b of the first and second holding members 62, 64.

The sectional shapes (outer shape) of the tubular electrode 122 is, for example, circular, but the outer shape of its cross section is permitted to have various shapes such as elliptic and polygonal shapes. The sectional shape of the grooves 92, 94 of the holding surfaces 82, 84 is preferred to be similar to the outer shape of the tubular electrode 122, but is permitted to be various shapes such as circular, elliptic and polygonal shapes. Moreover, the fluid (physiological saline) is not only discharged from the distal end of the tubular electrode 122 but also can be discharged from the side surface of the tubular electrode 122. That is, a large number of liquid dischargers (small holes) 122b having a diameter of, for example, 1 mm or less are formed in the tubular electrode 122. Thus, the fluid can be substantially uniformly supplied to the joint surfaces of the living tissues.

Now, the effects of a medical treatment apparatus 10 according to this embodiment are described with FIG. 18.

As described in the first embodiment, the steps from a first stage output to a fourth stage output are carried out. In this case, simultaneously with the switching of the circuits (S12) between the stopping of the third stage output (S11) and the start of the fourth stage output (S14), the tubular electrode 122 is moved backward (S33), instead of moving backward the rod electrode 66 described in the first embodiment (S13).

Moreover, in the present embodiment, the physiological saline is injected (S31) between the stopping of the first stage output (S3) and the start of the second stage output (S5). The physiological saline is also injected (S32) between the stopping of the second stage output (S7) and the start of the third stage output (S9). In addition, the physiological saline is discharged from the tubular electrode 122 through the conveying tube 114 and the lengthwise feed tube 46a by the operation of the unshown foot switch or hand switch connected to the flow volume adjuster 116 of the fluid feeding device 102. That is, the physiological saline is directly discharged to the joint surfaces of the target living tissues. The impedance Z of the living tissues between the electrodes 82b, 84b is dropped by the above-mentioned discharge of the physiological saline. More energy can be applied to the target living tissues by dropping the impedance Z. That is, proteins of the living tissues can be more easily denatured.

In addition, the discharge volume of the fluid is preset by, for example, an output controller 24 here.

The physiological saline can be discharged from the tubular electrode 122 before and after or simultaneously with the output of the tubular electrode 122. Similarly, the physiological saline can be discharged from the tubular electrode 122 before or simultaneously with the application of electricity to the living tissues between the electrodes 82b, 84b of the holding members 62, 64.

As described above, the following can be said according to this embodiment.

The rod electrode 66 alone is disposed on the joint surfaces of the living tissues held by the holding members 62, 64 in the first embodiment. One of the advantage is that not only the currents from the electrodes 82b, 84b of the holding members 62, 64 are applied but also a current is directly applied to the joint surfaces of the living tissues, such that the joint surfaces can be more denatured and dehydrated.

In the present embodiment, the tubular electrode 122 is provided which is capable of applying electricity to the living tissues between the first and second electrodes 82b, 84b and also capable of feeding a fluid to the joint surfaces of the held living tissues. Such a structure makes it possible to apply electricity to the joint surfaces of the living tissues and also inject a fluid thereto. Owing to the function of injecting a fluid to the joint surfaces, the impedance Z of the living tissues between the holding members 62, 64 can be dropped after the first stage output. Thus, more energy can be applied to the target living tissues. As a result, more energy can be applied to the joint surfaces.

In addition, although the conductive fluid (physiological saline) is fed to drop the impedance Z in this embodiment, a drug solution necessary to treat the living tissues, for example, may be fed.

Furthermore, although the application of electricity to the tubular electrode 122, the discharge of the fluid to the living tissues, the backward movement of the tubular electrode 122 and the application of electricity to the living tissues between the holding members 62, 64 are manually performed in the case described in this embodiment, the series of operations may be set to be automatically performed.

Still further, a plurality of tubular electrodes 122 can be arranged, similarly to the above-mentioned fourth and fifth electrodes.

### [Fourth Embodiment]

Next, a fourth embodiment is described with FIG. 19A to FIG. 19C. This embodiment is a modification of the first to third embodiments, and the same parts as the parts described in the first to third embodiments are provided with the same numerals and are not described in detail.

As shown in FIG. 19A to FIG. 19C, first and second holding members 62, 64 are structured to be able to discharge a fluid. A main body 62a of the first holding member 62 is provided with an electrode 132 having a planar surface to be in contact with living tissues. The electrode 132 is substantially rectangular, and an annular groove 134 serving as the passage of vapor generated from the living tissues is formed on the outer periphery of the electrode 132. On the other hand, a groove 92 where a rod electrode 66 and a tubular electrode 122 are disposed is formed on the central axis of the main body 62a of the first holding member 62, as described in the first to third embodiments.

A conduit 136 is provided within the main body 62a of the first holding member 62. The conduit 136 bends substantially in the shape of L within the main body 62a of the first holding member 62, and a plurality of openings (through-holes) 136a are formed in the conduit 136. The plurality of openings 136a are open in the surface of the electrode 132. That is, the conduit 136 is in communication with the outside of the electrode 132. In particular, the plurality of openings 136a are formed with the same diameter at predetermined intervals at positions predetermined distance away from the central axis of the main body 62a of the first holding member 62. Thus, when a fluid such as a physiological saline flows through the conduit 136, the fluid is discharged from the openings 136a of the conduit 136.

In addition, the openings 136a are provided at equal intervals at positions substantially parallel with the groove 92 formed on the central axis of the main body 62a of the first holding member 62. The outer peripheries of the openings 136a are covered with insulating materials. It is also preferable that the conduit 136 itself be formed of an insulating material. The conduit 136 is preferred to be, for example, a circularly cylindrical or squarely cylindrical, but is permitted to have various cross sectional shapes such as elliptically cylindrical or polygonally cylindrical shapes.

The conduit 136 is formed continuously to a handle 32 through a cylindrical member 42 of a shaft 34 or between the cylindrical member 42 and a sheath 44. The conduit 136 is in communication with a conveying tube 114, and enables the physiological saline fed from a bag 112 through the conveying tube 114 to be discharged not only from the tubular electrode 122 but also from the openings 136a provided inside the electrodes 132, 142 of the first and second holding members 62, 64.

The proximal end of the electrode 132 opposite to the side facing the second holding member 64 is connected to a cable 16 extending from the handle 32 via a first conducting line 18a.

Although not shown, the second holding member 64 is formed symmetrically to the first holding member 62. Here, for convenience of explanation, the numeral 142 is assigned to the electrode provided in the second holding member 64, the numeral 144 is assigned to an annular groove, the numeral 146 is assigned to a conduit, and the numeral 146a is assigned to openings.

In the case where the electrodes 132, 142 of the first and second holding members 62, 64 have the same potential (homopolar), living tissues in contact with the electrode 132 of the first holding member 62 and the electrode 142 of the second holding member 64 is heated when supplied with energy (high-frequency electric power) from an energy source 14. In this case, the electrodes 132, 142 serve as sensors to measure a current, a voltage, etc. flowing the electrodes 132, 142 through the living tissues. Then, the electrodes 132, 142 input relevant signals to a detecting portion 22 of the energy source 14 through the first and second conducting lines 18a, 18b.

In addition, the conduits 136, 146 of the first and second holding members 62, 64 extend to the handle 32 through the cylindrical member 42 of the shaft 34. These conduits 136, 146 extend from the handle 32 as tubes (not shown) provided side by side with the first and second conducting lines 18a, 18b, and are connected to, for example, the conveying tube 114 (see FIG. 15). Thus, a liquid such as a conductive fluid can be injected into the openings 136a, 146a through the conduits 136, 146.

The openings 136a, 146a are circular in FIG. 19A to FIG. 19C, but are not limited to the circular shape and are permitted to have various shapes such as elliptic and polygonal shapes. Further, the openings 136a in the first holding member 62 and the openings 146a in the second holding member 64 are not exclusively aligned at predetermined intervals along the longitudinal direction of the first and second high-frequency electrodes 132, 142, and are permitted to be arranged in a plurality of lines or at random.

### [Fifth Embodiment]

Next, a fifth embodiment is described with FIG. 20A to FIG. 21. This embodiment is a modification of the first to fourth embodiments, and the same parts as the parts described in the first to fourth embodiments are provided with the same numerals and are not described in detail.

As shown in FIG. 20A to FIG. 20C, an energy treatment instrument 12 in the present embodiment is provided with the mechanism capable of separating the rod electrode 66 described in the second embodiment, in addition to the mechanism capable of moving the tubular electrode 122 described in the third embodiment. That is, the energy treatment instrument 12 according to the present embodiment is provided with to a mechanism capable of separating the tubular electrode 122.

The mechanism capable of separating the tubular electrode 122 shown in FIG. 20A to FIG. 20C is about the same as the mechanism capable of separating the rod electrode 66 described in the second embodiment. A lengthwise feed tube 46a and a connecting tube 46c are provided inside a cylindrical member 42 of a shaft 34. In this embodiment, in order to carry a fluid, the connecting tube 46c is provided instead of the connection rod 46b. Moreover, instead of the rod electrode 66 in the second embodiment, the tubular electrode 122 is disposed at the distal ends of the lengthwise feed tube 46a and the connecting tube 46c.

In addition, the distal end of a third conducting line 18c is fixed inside the distal end of the connecting tube 46c, and extends to the side of a handle 32.

The effects of a medical treatment apparatus 10 are shown in a flowchart in FIG. 21. This flowchart is a combination of the flow described with FIG. 13 in the second embodiment and the flow described with FIG. 18 in the third embodiment.

### [Sixth Embodiment]

A sixth embodiment is described with FIG. 22 and FIG. 23. This embodiment is a modification of the third to fifth embodiments, and the same parts as the parts described in the third to fifth embodiments are provided with the same numerals and are not described in detail.

As shown in FIG. 22, a liquid discharging nozzle 152 having a flow path 152a is disposed here instead of the tubular electrode 122. Therefore, as shown in FIG. 22, no third conducting line 18c (see FIG. 2 and FIG. 16) is connected to the nozzle 152. That is, the third conducting line 18c is removed from a cable 16. Thus, electrodes 82b, 84b provided in first and second holding members 62, 64 are used to join living tissues together. Moreover, although a switching unit 28 is provided in this embodiment as shown in FIG. 22, there is no need to switch circuits in this embodiment. That is, no switching unit 28 may be provided.

Therefore, in this embodiment, a first treating portion 52 of a treatment portion 36 of an energy treatment instrument 12 includes the pair of holding members 62, 64 which can be opened and closed, and a second treating portion 54 includes the liquid discharging nozzle 152. The liquid discharging nozzle 152 can discharge a fluid (physiological saline) not only from the distal end of the liquid discharging nozzle 152 but also from the side surface of the liquid discharging nozzle 152. That is, a large number of liquid discharging holes 152b having a diameter of, for example, 1 mm or less are formed in the liquid discharging nozzle 152. Thus, the fluid can be substantially uniformly supplied to the joint surfaces of the living tissues.

A second knob 32b (see FIG. 15) provided in a handle 32 functions as forward/backward movement knob for the liquid discharging nozzle 152. The second knob 32b is connected to, for example, the outer wall of a conveying tube 114 inside the handle 32. If the second knob 32b is moved toward the operator, the conveying tube 114 moves toward the operator. Then, the liquid discharging nozzle 152 connected to the distal end of the conveying tube 114 moves toward the operator, and is stored within the shaft 34.

Now, the effects of a medical treatment apparatus 10 according to this embodiment are described with FIG. 23.

Living tissues are held between holding surfaces 82, 84 of the first and second holding members 62, 64. At the same time, the liquid discharging nozzle 152 is disposed between the joint surfaces of the living tissues. That is, the liquid discharging nozzle 152 is held between the living tissues.

In this state, a foot switch or hand switch connected to an energy source 14 is operated. Electricity is applied from the energy source 14 to the living tissues between the first electrode 82b and the second electrode 84b of the first and second holding members 62, 64 (S41). Thus, proteins of the living tissues held by the holding members 62, 64 are denatured, and the living tissues are dehydrated simultaneously. At the same time, the impedance Z is calculated by a detecting portion 22 of the energy source 14 through the electrodes 82b, 84b.

Whether the calculated impedance Z has exceeded, for example, 500 [Ω] (not limited to this value and any value can be set) set as a threshold value in an output controller 24 is judged (S42). In this case, a lower impedance Z is set so that water contained in the target living tissues may not be completely removed and the living tissues may be sufficiently denatured.

When the impedance Z is judged to have exceeded a threshold value of 500 [Ω] in accordance with the detection by the detecting portion 22, the output controller 24 stops the output of high-frequency electric power from an output unit 26 (S43).

Then, if the foot switch or hand switch connected to a flow volume adjuster 116 of a fluid feeding device 102 is operated, the physiological saline is discharged from the liquid discharging nozzle 152 through the conveying tube 114 (S44). Since a large number of liquid discharging holes 152b are formed in the nozzle 152, the physiological saline is discharged to the joint surfaces of the target living tissues. If the foot switch or hand switch is operated (released) or if a predetermined flow volume of physiological saline has been discharged, the discharge of the physiological saline is stopped. The discharge volume can be preset by, for example, the flow volume adjuster 116.

Then, the distal end of the liquid discharging nozzle 152 disposed in close contact with the joint surfaces is moved backward into the shaft 34 (S45). At the same time, the electrodes 82b, 84b disposed in grooves 92, 94 of the first and second holding members 62, 64 are urged by elastic members 92a, 94a (see

FIG. 20A to FIG. 20C) provided inside main bodies 62a, 64a of the first and second holding members 62, 64 so that the joint surfaces of the living tissues may be in close contact with each other.

The foot switch or hand switch connected to the energy source 14 is again operated with a stop condition (threshold value) of an impedance of, for example, 1000 [Ω]. Electricity is applied from the energy source 14 to the living tissues between the first electrode 82b and the second electrode 84b of the first and second holding members 62, 64 (S46). Thus, proteins of the living tissues held by the holding members 62, 64 are denatured, and the living tissues are dehydrated simultaneously. At the same time, the impedance Z is calculated by the detecting portion 22 of the energy source 14 through the electrodes 82b, 84b.

Then, the joint surfaces of the tissues are completely dehydrated by an output with an impedance of, for example, 1000 [Ω] as a stop condition (threshold value) (S47). That is, whether the impedance Z detected by the detecting portion 22 reaches 1000 [Ω] is judged, and the output is stopped when the impedance has reached 1000 [Ω] (S48). Thus, parts where the liquid discharging nozzle 152 has been disposed are joined together by the action of high-frequency energy. Therefore, the high-frequency energy acts on the joint surfaces to which the fluid has been injected to facilitate the application of the energy, such that the joint surfaces can be denatured and dehydrated. Consequently, the living tissues can be joined together.

Although the first output (S41 to S43), the injection of the physiological saline (S44) and the second output (S46 to S48) are manually performed here, the series of operations may be set to be automatically performed.

The physiological saline can be discharged from the liquid discharging nozzle 152 before or simultaneously with the output of the electrodes 82b, 84b of the holding members 62, 64. In this case, the liquid discharging nozzle 152 is moved backward after the second output has been stopped.

The following can be said according to this embodiment.

The rod electrode 66 is disposed on the joint surfaces of the held living tissues in the first embodiment. The advantage is that not only the currents from the electrodes 82b, 84b of the holding members 62, 64 are applied but also a current is directly applied to the joint surfaces of the living tissues, such that the joint surfaces are more denatured and dehydrated.

In the present embodiment, the liquid discharging nozzle 152 is provided which can inject a physiological saline to the joint surfaces of the held living tissues. This structure makes it possible to drop the impedance Z of the joint surfaces by the injection of the physiological saline after electricity has been applied to the whole target living tissues held by the first and second holding members 62, 64. Therefore, electricity can be again applied to the joint surfaces of the living tissues. As a result, more energy can be applied to the joint surfaces, and the living tissues can be more firmly joined together.

### [Seventh Embodiment]

Next, a seventh embodiment is described with FIG. 24A to FIG. 24C. This embodiment is a modification of the sixth embodiment, and the same parts as the parts described in the sixth embodiment are provided with the same numerals and are not described in detail.

In the present embodiment, as shown in FIG. 24A to FIG. 24C, a separating mechanism is provided in addition to the forward/backward movement mechanism for the liquid discharging nozzle 152 described in the sixth embodiment.

The separating mechanism is the same as the separating mechanisms described in the second to fifth embodiments and is not described. The rod electrode 66 in the second embodiment is replaced with the liquid discharging nozzle 152 in the present embodiment.

Additional advantages and modifications will readily occur to those skilled in the art. Therefore, the invention in its broader aspects is not limited to the specific details and representative embodiments shown and described herein. Accordingly, various modifications may be made without departing from the spirit or scope of the general inventive concept as defined by the appended claims and their equivalents.

## Claims

1. A medical treatment apparatus (10) to join target living tissues in a body, the medical treatment apparatus **characterized by** comprising:
an energy source (14) which applies energy to the target living tissues;
a first treating portion (52) which joins the target living tissues together when energy is applied thereto from the energy source, the first treating portion including:
at least a pair of holding members (62, 64) having holding surfaces to hold the target living tissues; and
an energy output unit (82b, 84b) which is provided on the holding surfaces of the holding members and which join the target living tissues together when energy is applied thereto from the energy source;
a second treating portion (54) which is interposed between the target living tissues and which denatures a joint surface of the target living tissues in contact with the second treating portion;
an operation portion (32) having a function of operating the holding members so that at least one of the holding members moves relative to the other;
a detecting portion (22) which detects at least one of biological information regarding the living tissues held by the pair of holding members and biological information regarding the living tissues between the pair of holding members and the second treating portion; and
a controller (24) which controls an output of the energy output unit and the second treating portion on the basis of the biological information regarding the living tissues obtained in the detecting portion.

2. The medical treatment apparatus (10) according to claim 1, **characterized in that** the energy output unit (82b, 84b) has electrodes to supply energy to the target living tissues.

3. The medical treatment apparatus (10) according to claim 1, **characterized in that** the second treating portion (54) has an electrode (66) which is connected to the energy source and which supplies energy to the target living tissues.

4. The medical treatment apparatus (10) according to claim 1, **characterized in that** the controller (24) controls the output of the energy output unit (82b, 84b) and the second treating portion (66) on the basis of at least one kind of biological information detected by the detecting portion (22) selected from the group consisting of a current flowing through the target living tissues, a voltage between the energy output unit or between the energy output unit and the second treating portion, impedance (Z) of the target living tissues, and phase difference information.

5. The medical treatment apparatus (10) according to claim 1, **characterized in that**
the holding member (62, 64) has a distal end, a proximal end and a longitudinal direction determined by the distal end and proximal end, and
the second treating portion (54) is able to move in the longitudinal direction of the holding member determined by the distal end and proximal end.

6. The medical treatment apparatus (10) according to claim 1, **characterized in that** at least one of the holding members (62, 64) has an elastic member (92a, 94a) which applies pressure to the target living tissues by the energy output unit (82b, 84b) when the second treating portion (54) moves backward toward the proximal end of the holding member.

7. The medical treatment apparatus (10) according to claim 1, **characterized in that** the second treating portion (54) has an ultrasonic probe which oscillates ultrasonic vibration.

8. The medical treatment apparatus (10) according to claim 1, **characterized in that** the second treating portion (54) has a hollow flow path (122a; 152a), and at least one opening (122b; 152b) which is in communication with the flow path and which is formed in the outer surface of the second treating portion.

9. The medical treatment apparatus (10) according to claim 1, **characterized by** further comprising a connecting member (46a, 46b) which connects the operation portion (32) and the second treating portion (54),
wherein the second treating portion is removably attached to and detached from the connecting member and to remain interposed between the target living tissues.

10. The medical treatment apparatus (10) according to claim 1, **characterized in that** the energy output unit (82b, 84b) applies energy to the target living tissues by at least one of high-frequency energy, and heat energy from resistance heating.

11. The medical treatment apparatus (10) according to claim 1, **characterized in that** the second treating portion (54) applies energy to the target living tissues by at least one of high-frequency energy, ultrasonic energy, and heat energy from resistance heating.

12. A medical treatment instrument (12) to join target living tissues in a body, the treatment instrument **characterized by** comprising:
a first treating portion (52) which joins the target living tissues together when energy is applied thereto from an energy source (14), the first treating portion including:
at least a pair of holding members (62, 64) having holding surfaces (82, 84) to hold the target living tissues; and
an energy output unit (82b, 84b) which is provided on the holding surfaces of the holding members and which join the target living tissues together when energy is applied thereto from the energy source;
a second treating portion (54) which is interposed between the target living tissues and which denatures the surfaces of the target living tissues in contact with the second treating portion; and
an operation portion (32) which has a function of operating the holding members so that at least one of the holding members moves relative to the other,
wherein the energy output unit and the second treating portion are able to detect at least one of biological information regarding the living tissues held by the pair of holding members and biological information regarding the living tissues between the holding member and the second treating portion, and control energy output from the energy source to the energy output unit and the second treating portion is controlled on the basis of the biological information regarding the living tissues obtained by the energy output unit and the second treating portion.

13. The medical treatment instrument (12) according to claim 12, **characterized in that** the energy output unit (82b, 84b) applies energy to the target living tissues by at least one of high-frequency energy, and heat energy from resistance heating.

14. The medical treatment instrument (12) according to claim 12, **characterized in that** the second treating portion (54) applies energy to the target living tissues by at least one of high-frequency energy, ultrasonic energy, and heat energy from resistance heating.

15. The medical treatment instrument (12) according to claim 12, **characterized in that**
the holding member (62, 64) has a distal end, a proximal end and a longitudinal direction of the holding member determined by the distal end and proximal end, and
the second treating portion (54) is able to move in the longitudinal direction of the holding member determined by the distal end and proximal end.

16. The medical treatment instrument (12) according to claim 12, **characterized in that** at least one of the holding members (62, 64) has an elastic member (92a, 94a) which applies pressure to the target living tissues by the energy output unit (82b, 84b) when the second treating portion (54) moves backward toward the proximal end of the holding member.

17. The medical treatment instrument (12) according to claim 12, **characterized in that** the second treating portion (54) has a hollow flow path (122a; 152a), and at least one opening (122b; 152b) which is in communication with the flow path and which is formed in the outer surface of the second treating portion.

18. The medical treatment instrument (12) according to claim 12, **characterized by** further comprising a connecting member (46a, 46b) which connects the operation portion (32) and the second treating portion (54),
wherein the second treating portion (54) is removably attached to and detached from the connecting member and to remain interposed between the target living tissues.
